# EUROPEAN PATENT APPLICATION

(11) **EP 2 827 166 A1**
(43) Date of publication of application: **21.01.2015**
(21) Application number: 14177322.6
(22) Date of filing: 16.07.2014
(51) Int. Cl.: G01R 33/36, G01R 33/54

(54) **Magnetic resonance imaging apparatus and notification information providing method performed by using the same and radio frequency coil and notification information providing method performed by using the radio frequency coil**

(30) Priority: 17.07.2013 KR 20130084382
(71) Applicant: Samsung Electronics Co., Ltd, Gyeonggi-do 443-742 (KR)
(72) Inventor: Han, Jeong-Ho, Seoul (KR); Ban, Dae-hyun, Seoul (KR); Oh, Keum-yong, Gyeonggi-do (KR)
(74) Representative: Lubberdink, Pim

(57) **Abstract**

A notification method performed by using a magnetic resonance imaging (MRI) apparatus includes: obtaining information regarding a first radio frequency (RF) coil for photographing an object; obtaining information regarding a second RF coil connected to the MRI apparatus; comparing the information regarding the first RF coil and the information regarding the second RF coil; and outputting notification information indicating that the second RF coil corresponds to the first RF coil based on a result of the comparing. In one embodiment, the first RF coil is the one selected by the user for a given MRI scan and the user is notified by a green light in case the second RF coil connected to the MRI system corresponds to the selected RF coil. In case the connected RF coil is different from the connected one, a red light is provided.

## Description

### RELATED APPLICATIONS

This application claims the benefit of Korean Patent Application No. 10-2013-0084382, filed on July 17, 2013, in the Korean Intellectual Property Office, the disclosure of which is incorporated herein in its entirety by reference.

### BACKGROUND

### 1. Field

One or more embodiments relate to a magnetic resonance imaging (MRI) apparatus and a notification information providing method performed by using the MRI apparatus, and radio frequency (RF) coils and a notification information providing method performed by using the RF coils.

### 2. Description of the Related Art

A magnetic resonance imaging (MRI) apparatus is an apparatus for acquiring a tomographic image of a part of an object by expressing, in a contrast comparison, a strength of a MR signal with respect to a radio frequency (RF) signal generated in a magnetic field having a specific strength. For example, if an RF signal that resonates only a specific atomic nucleus (for example, a hydrogen atomic nucleus) is irradiated for an instant onto the object that is placed in a strong magnetic field and then such irradiation stops, an MR signal is emitted from the specific atomic nucleus, and thus the MRI apparatus may receive the MR signal and acquire an MR image. An intensity of the MR signal may be determined according to concentration of a predetermined atom (for example, hydrogen) included in the object, a relaxation time T1, a relaxation time T2, and a blood flow.

MRI apparatuses include characteristics different from those of other imaging apparatuses. Unlike image apparatuses such as computed tomography (CT) apparatuses that acquire images dependent upon a direction of detection hardware, MRI apparatuses may acquire two-dimensional (2D) images or three-dimensional (3D) volume images that are oriented toward an optional point. MRI apparatuses do not expose radiation to objects and examinees, unlike CT apparatuses, X-ray apparatuses, position emission tomography (PET) apparatuses, and single photon emission CT (SPECT) apparatuses, may acquire images having high soft tissue contrast, and may acquire neurological images, intravascular images, musculoskeletal images, and oncologic images that are important to precisely describe abnormal tissues.

### SUMMARY

One or more exemplary embodiments include a magnetic resonance imaging (MRI) apparatus and a notification information providing method performed by using the MRI apparatus, and radio frequency (RF) coils and a notification information providing method performed by using the RF coils whereby user convenience may increase when an object is photographed.

One or more exemplary embodiments include an MRI apparatus and a notification information providing method performed by using the MRI apparatus and RF coils and a notification information providing method performed by using the RF coils whereby an object may be safely photographed and damage to the RF coil may be prevented.

Additional aspects will be set forth in part in the description which follows and, in part, will be apparent from the description, or may be learned by practice of the presented embodiments.

According to one or more exemplary embodiments, there is provided a notification information providing method performed by using a magnetic resonance imaging (MRI) apparatus, the notification information providing method including: obtaining information regarding a first radio frequency (RF) coil for photographing an object; obtaining information regarding a second RF coil connected to the MRI apparatus; comparing the information regarding the first RF coil and the information regarding the second RF coil; and outputting notification information indicating that the second RF coil corresponds to the first RF coil based on a result of the comparing.

The outputting may include: outputting the notification information by using at least one of a speaker and a display that are attached to a gantry of the MRI apparatus or the second RF coil.

The second RF coil may include a wired RF coil connected to the MRI apparatus via a connector.

The outputting may include: transmitting a light signal having a color corresponding to the notification information to the second RF coil via the connector and displaying the color on a display attached to the second RF coil.

The obtaining of the information regarding the second RF coil may include: measuring a resistance value of the connector; and obtaining the information regarding the second RF coil based on the measured resistance value.

The obtaining of the information regarding the second RF coil may include: receiving the information regarding the second RF coil from the second RF coil via the connector.

The second RF coil may be connected to the MRI apparatus by wire or by using a first wireless communication method, the method may further include: when the second RF coil does not correspond to the first RF coil, transmitting the information regarding the first RF coil to a plurality of RF coils that are not connected to the MRI apparatus by using a second wireless communication method.

The information regarding the first RF coil and the information regarding the second RF coil may include information regarding at least one of parts in which the first and second RF coils are installed, sizes, a number of channels, and use.

According to one or more exemplary embodiments, there is provided a non-transitory computer-readable storage medium storing a computer program for executing the notification information providing method.

According to one or more exemplary embodiments, there is provided a MRI apparatus including: an information obtaining unit for obtaining information regarding a first RF coil for photographing an object and obtaining information regarding a second RF coil connected to the MRI apparatus; a comparison unit for comparing the information regarding the first RF coil and the information regarding the second RF coil; and an output unit for outputting notification information indicating that the second RF coil corresponds to the first RF coil based on a result of the comparing.

The output unit may output the notification information by using at least one of a speaker and a display that are attached to a gantry of the MRI apparatus or the second RF coil.

The second RF coil may include a wired RF coil connected to the MRI apparatus via a connector.

The output unit may transmit a light signal having a color corresponding to the notification information to the second RF coil via the connector and display the color on a display attached to the second RF coil.

The information obtaining unit may measure a resistance value of the connector and obtain the information regarding the second RF coil based on the measured resistance value.

The information obtaining unit may receive the information regarding the second RF coil from the second RF coil via the connector.

The second RF coil may be connected to the MRI apparatus by wire or by using a first wireless communication method, the apparatus further including: when the second RF coil does not correspond to the first RF coil, a communication unit for transmitting the information regarding the first RF coil to a plurality of RF coils that are not connected to the MRI apparatus by using a second wireless communication method.

According to one or more exemplary embodiments, there is provided a notification information providing method performed by using a RF coil that is not connected to a MRI apparatus, the notification information providing method including: receiving information regarding a predetermined RF coil for photographing an object from the MRI apparatus; comparing the information regarding the predetermined RF coil and information regarding the RF coil; and when the RF coil corresponds to the predetermined RF coil, outputting notification information indicating that the RF coil corresponds to the predetermined RF coil.

According to one or more exemplary embodiments, there is provided a RF coil that is not connected to an MRI apparatus, the RF coil including: a communication unit for receiving information regarding a predetermined RF coil for photographing an object from the MRI apparatus; a comparison unit for comparing the information regarding the predetermined RF coil and information regarding the RF coil; and an output unit for, when the RF coil corresponds to the predetermined RF coil, outputting notification information indicating that the RF coil corresponds to the predetermined RF coil.

According to one or more exemplary embodiments, there is provided a notification information providing method including: transmitting a first signal to a RF coil that is connected to an MRI apparatus via a connector by wire; determining a state in which the RF coil is installed according to whether a second signal corresponding to the first signal is received from the RF coil; and when the RF coil is abnormally installed in the MRI apparatus, outputting notification information as guidance indicating that the connector is to be separated from the MRI apparatus or the RF coil is to be normally installed in the MRI apparatus.

The outputting may include: outputting at least one of an alarm sound and an alarm screen as the notification information.

The notification information providing method may further include: determining whether the MRI apparatus photographs an object, wherein the transmitting of the first signal includes: transmitting the first signal to the RF coil only when the object is not photographed.

The transmitting of the first signal may include: transmitting the first signal along a first signal connection path connected to the RF coil, and wherein the determining of the state in which the RF coil is installed includes: determining whether the second signal is received via a second signal connection path connected to the RF coil.

The first signal connection path and the second signal connection path may include optical fiber or electrical conductive wires.

The RF coil may move in a first direction when the RF coil contacts the MRI apparatus or the object, and include a switch used to connect the first signal connection path and the second signal connection path when the RF coil moves in the first direction, and wherein the determining of the state in which the RF coil is installed includes: when the second signal is received from the RF coil by connecting the first signal connection path and the second signal connection path, determining that the RF coil is normally installed in the MRI apparatus.

The switch may include: a signal penetrating unit for penetrating the first signal; and a signal blocking unit disposed on the signal penetrating unit and blocking the first signal, wherein the signal penetrating unit is disposed between the first signal connection path and the second signal connection path when the switch moves in the first direction.

The determining of the state in which the RF coil is installed may include: when the second signal is not received from the RF coil due to the first signal connection path and the second signal connection path not being connected, determining that the RF coil is abnormally installed in the MRI apparatus.

The switch may include a plurality of switches, and wherein the first signal connection path and the second signal connection path include a plurality of first signal connection paths and a plurality of second signal connection paths that may be connected to each other when each of the plurality of switches moves in the first direction.

The transmitting of the first signal may include: transmitting the first signal to the RF coil along each of the plurality of first signal connection paths, wherein the determining of the state in which the RF coil is installed includes: determining whether the second signal is received from the RF coil via each of the plurality of second signal connection paths, and wherein the outputting includes: outputting location information of a switch corresponding to a second signal connection path via which the second signal is not received, from among the plurality of second signal connection paths.

The first signal connection path and the second signal connection path may not be connected to each other in the RF coil and extend to a contact unit of the RF coil that contacts a signal reflecting unit of the MRI apparatus, when the contact unit contacts the signal reflecting unit, the second signal flows along the second signal connection path by reflecting the first signal flowing along the first signal connection path by the signal reflecting unit, and wherein the determining of the state in which the RF coil is installed includes: when the second signal is received from the RF coil by reflecting the first signal by the signal reflecting unit, determining that the RF coil is normally installed in the MRI apparatus.

The outputting may include: outputting the notification information by using at least one of a speaker and a display that are attached to a gantry of the MRI apparatus or the RF coil.

According to one or more exemplary embodiments, there is provided a non-transitory computer-readable storage medium storing a computer program for executing the notification information providing method.

According to one or more exemplary embodiments, there is provided a MRI apparatus including: a signal transceiving unit for transmitting a first signal to a RF coil that is connected to the MRI apparatus via a connector by wire; a determining unit for determining a state in which the RF coil is installed according to whether the signal transceiving unit receives a second signal corresponding to the first signal from the RF coil; and an output unit for, when the RF coil is abnormally installed in the MRI apparatus, outputting notification information as guidance indicating that the connector is to be separated from the MRI apparatus or the RF coil is to be normally installed in the MRI apparatus.

The output unit may output at least one of an alarm sound and an alarm screen as the notification information.

The determining unit may determine whether the MRI apparatus photographs an object, wherein the signal transceiving unit transmits the first signal to the RF coil only when the object is not photographed.

The signal transceiving unit may transmit the first signal along a first signal connection path connected to the RF coil, and wherein the determining unit determines whether the signal transceiving unit receives the second signal via a second signal connection path connected to the RF coil.

The first signal connection path and the second signal connection path may include optical fiber or electrical conductive wires.

The RF coil may move in a first direction when the RF coil contacts the MRI apparatus or the object, and include a switch used to connect the first signal connection path and the second signal connection path when the RF coil moves in the first direction, and wherein the determining unit determines that the RF coil is normally installed in the MRI apparatus when the second signal is received from the RF coil by connecting the first signal connection path and the second signal connection path.

The switch may include: a signal penetrating unit for penetrating the first signal; and a signal blocking uniform disposed on the signal penetrating unit and blocking the first signal, wherein the signal penetrating unit is disposed between the first signal connection path and the second signal connection path when the switch moves in the first direction.

The determining unit may determine that the RF coil is abnormally installed in the MRI apparatus when the second signal is not received from the RF coil due to the first signal connection path and the second signal connection path not being connected.

The switch may include a plurality of switches, and wherein the first signal connection path and the second signal connection path include a plurality of first signal connection paths and a plurality of second signal connection paths that may be connected to each other when each of the plurality of switches moves in the first direction.

The signal transceiving unit may transmit the first signal to the RF coil along each of the plurality of first signal connection paths, wherein the determining unit determines whether the signal transceiving unit receives the second signal from the RF coil via each of the plurality of second signal connection paths, and wherein the output unit outputs location information of a switch corresponding to a second signal connection path via which the second signal is not received, from among the plurality of second signal connection paths.

The first signal connection path and the second signal connection path may not be connected to each other in the RF coil and extend to a contact unit of the RF coil that contacts a signal reflecting unit of the MRI apparatus, the MRI apparatus further including: a signal reflecting unit for allowing the second signal to flow along the second signal connection path by reflecting the first signal flowing along the first signal connection path when the contact unit contacts the MRI apparatus, and wherein the determining unit determines that the RF coil is normally installed in the MRI apparatus when the second signal is received from the RF coil by reflecting the first signal by the signal reflecting unit.

The outputting unit may output the notification information by using at least one of a speaker and a display that are attached to a gantry of the MRI apparatus or the RF coil.

According to one or more exemplary embodiments, there is provided a RF coil installed in an MRI apparatus, the RF coil including: a first signal connection path along which a first signal flows; a second signal connection path along which a second signal corresponding to the first signal flows; and a switch for moving in a first direction when the RF coil contacts the MRI apparatus or an object, wherein the switch connects the first signal connection path and the second signal connection path when the switch moves in the first direction so that the second signal corresponding to the first signal flowing along the first signal connection path may flow along the second signal connection path.

The first signal connection path may be connected to the MRI apparatus and allows the first signal output from the MRI apparatus to flow, and wherein the second signal connection path is connected to the MRI apparatus and allows the second signal to flow to the MRI apparatus.

The RF coil may further include: a signal transceiving unit for transmitting the first signal along the first signal connection path and receiving the second signal via the second signal connection path.

The RF coil may further include: a determining unit for determining a state in which the RF coil is installed according to whether the signal transceiving unit receives the second signal flowing along the second signal connection path; and an output unit for, when the RF coil is abnormally installed in the MRI apparatus, outputting notification information as guidance indicating that the RF coil is to be normally installed in the MRI apparatus.

The switch may include a plurality of switches, and wherein the first signal connection path and the second signal connection path include a plurality of first signal connection paths and a plurality of second signal connection paths that may be connected to each other when each of the plurality of switches moves in the first direction.

The signal transceiving unit may transmit the first signal along each of the plurality of first signal connection paths, wherein the determining unit determines whether the signal transceiving unit receives the second signal via each of the plurality of second signal connection paths, and wherein the output unit outputs location information of a switch corresponding to a second signal connection path via which the signal transceiving unit does not receive the second signal, from among the plurality of second signal connection paths.

According to one or more exemplary embodiments, there is provided a RF coil installed in an MRI apparatus, the RF coil including: a first signal connection path along which a first signal flows; a second signal connection path along which a second signal corresponding to the first signal flows; and a contact unit for contacting a signal reflecting unit of the MRI apparatus, wherein the first signal connection path and the second signal connection path are not connected to each other and extend to the contact unit, and wherein the contact unit contacts the signal reflecting unit so that the first signal flowing along the first signal connection path is reflected by the signal reflecting unit and the second signal corresponding to the first signal flows along the second signal connection path.

The first signal connection path may be connected to the MRI apparatus and allows the first signal output from the MRI apparatus to flow, and wherein the second signal connection path is connected to the MRI apparatus and allows the second signal to flow to the MRI apparatus.

The RF coil may further include: a signal transceiving unit for transmitting the first signal along the first signal connection path and receiving the second signal via the second signal connection path.

The first signal connection path and the second signal connection path may include optical fiber or electrical conductive wires.

### BRIEF DESCRIPTION OF THE DRAWINGS

These and/or other aspects will become apparent and more readily appreciated from the following description of the exemplary embodiments, taken in conjunction with the accompanying drawings of which:
FIG. 1 is a block diagram of a magnetic resonance imaging (MRI) apparatus and radio frequency (RF) coils that may be connected to the MRI apparatus according to an exemplary embodiment;
FIG. 2 is a flowchart of a notification information providing method performed by using an MRI apparatus according to an exemplary embodiment;
FIG. 3 is a diagram of a state in which a RF coil is connected to an MRI apparatus;
FIG. 4 is a flowchart of a notification information providing method performed by using an MRI apparatus according to another exemplary embodiment;
FIG. 5 is a flowchart of a notification information providing method performed by using an MRI apparatus according to another exemplary embodiment;
FIG. 6A is a block diagram illustrating a configuration of an MRI apparatus according to an exemplary embodiment, and FIG. 6B is a block diagram illustrating a configuration of an MRI apparatus according to another exemplary embodiment;
FIG. 7 is a flowchart of a notification information providing method performed by using a RF coil according to an exemplary embodiment;
FIG. 8 is a block diagram illustrating a configuration of a RF coil according to an exemplary embodiment;
FIG. 9 is a flowchart of a notification information providing method performed by using an MRI apparatus according to another exemplary embodiment;
FIG. 10 is an exemplary diagram of a RF coil that is to be installed in an MRI apparatus according to an exemplary embodiment;
FIG. 11A is a lateral view of the RF coil of FIG. 10, and FIG. 11B is a diagram of a switch of FIG. 11A that is moved in a first direction;
FIG. 12 is an exemplary diagram of a RF coil that is to be installed in an MRI apparatus according to another exemplary embodiment;
FIG. 13 is a flowchart of a notification information providing method performed by using an MRI apparatus according to another exemplary embodiment;
FIG. 14 is an exemplary diagram of a RF coil that is to be installed in an MRI apparatus according to another exemplary embodiment;
FIG. 15A is a diagram of a head RF coil including a switch, and FIG. 15B is a diagram of a spine RF coil including the switch;
FIG. 16A is an exemplary diagram of a RF coil that is to be installed in an MRI apparatus according to another exemplary embodiment, and FIG. 16B is a diagram of a signal reflecting unit and a contact unit of FIG. 16A that contact each other;
FIG. 17 is a block diagram illustrating a configuration of an MRI apparatus according to another exemplary embodiment;
FIG. 18 is a block diagram illustrating a configuration of a RF coil according to another exemplary embodiment;
FIG. 19 is a block diagram illustrating a configuration of a RF coil according to another exemplary embodiment;
FIG. 20 is a block diagram illustrating a configuration of an MRI apparatus according to another exemplary embodiment; and
FIG. 21 is a diagram illustrating a configuration of a communication unit included in the MRI apparatus of FIG. 20.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the exemplary embodiments are merely described below, by referring to the figures, to explain aspects of the present description.

The advantages, features and aspects of the present invention will become apparent from the following description of the exemplary embodiments with reference to the accompanying drawings, which is set forth hereinafter. The present invention may, however, be embodied in different forms and should not be construed as limited to the exemplary embodiments set forth herein. Rather, these embodiments are provided so that this disclosure will be thorough and complete, and will fully convey the scope of the present invention to those of ordinary skill in the art.

Terms used herein will be briefly described, and the present invention will be described in detail.

Terms used in the present invention have been selected as general terms which are widely used at present, in consideration of the functions of the present invention, but may be altered according to the intent of an operator skilled in the art, conventional practice, or introduction of new technology. Also, if there is a term which is arbitrarily selected by the applicant in a specific case, in which case a meaning of the term will be described in detail in a corresponding description portion of the present invention. Therefore, the terms should be defined on the basis of the entire content of this specification instead of a simple name of each of the terms.

In this disclosure below, when it is described that one comprises (or includes or has) some elements, it should be understood that it may comprise (or include or has) only those elements, or it may comprise (or include or have) other elements as well as those elements if there is no specific limitation. The term "module", as used herein, means, but is not limited to, a software or hardware component, such as a Field Programmable Gate Array (FPGA) or an Application Specific Integrated Circuit (ASIC), which performs certain tasks. A module may advantageously be configured to reside in an addressable storage medium and configured to execute on one or more processors. Thus, a module may include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. The functionality provided for in the components and modules may be combined into fewer components and modules or further separated into additional components and modules.

The term "at least one of A and B" is used herein to include selection of A, selection of B, or selection of A and B. As an additional example, the term "at least one of A, B, and C" may include selection of A, selection of B, selection of C, selection of A and B, selection of B and C, or selection of A, B, and C. A list of more items may be obviously extended and interpreted by those of ordinary skill in the art.

The term "image" used herein may denote multi-dimensional data composed of discrete image factors (for example, pixels in a two-dimensional (2D) image and pixels in a three-dimensional (3D) image). For example, an image may include a medical image of an object which is acquired by an X-ray apparatus, a computed tomography (CT) apparatus, a magnetic resonance imaging (MRI) apparatus, an ultrasonic apparatus, or another medical image photographing apparatus.

Moreover, the term "object" used herein may include a person, an animal, a part of the person, or a part of the animal. For example, an object may include an organ such as a liver, a heart, a womb, a brain, breasts, an abdomen, or the like, or a blood vessel. Also, the term "object" may include a phantom. The phantom denotes a material having a volume very close to a density of organisms and an effective atomic number, and may include a spherical phantom having characteristics similar to those of a human body.

The "user" used herein is a medical expert, and may be a doctor, a nurse, a medical technologist, a medical image expert, or the like, or may be an engineer repairing a medical apparatus. However, the user is not limited thereto.

The magnetic resonance imaging (MRI) used herein is an image of an object obtained by using a nuclear MR principle.

The "pulse sequence" used herein means continuation of repeatedly applied signals in an MRI apparatus. The pulse sequence may include a time parameter of an RF pulse, for example, a repetition time TR and a time to echo TE.

The "schematic pulse sequence view" used herein shows a sequence of events that occur in the MRI apparatus. For example, the schematic pulse sequence view may be a schematic view of a radio frequency (RF) pulse, a gradient magnetic field, an MR signal, etc.

Exemplary embodiments of the present invention capable of being easily embodied by those of ordinary skill in the art will now be described in detail with reference to the accompanying drawings. In the accompanying drawings, a portion irrelevant to a description of the present invention will be omitted for clarity. As used herein, the term "and/or" includes any and all combinations of one or more of the associated listed items. Expressions such as "at least one of," when preceding a list of elements, modify the entire list of elements and do not modify the individual elements of the list.

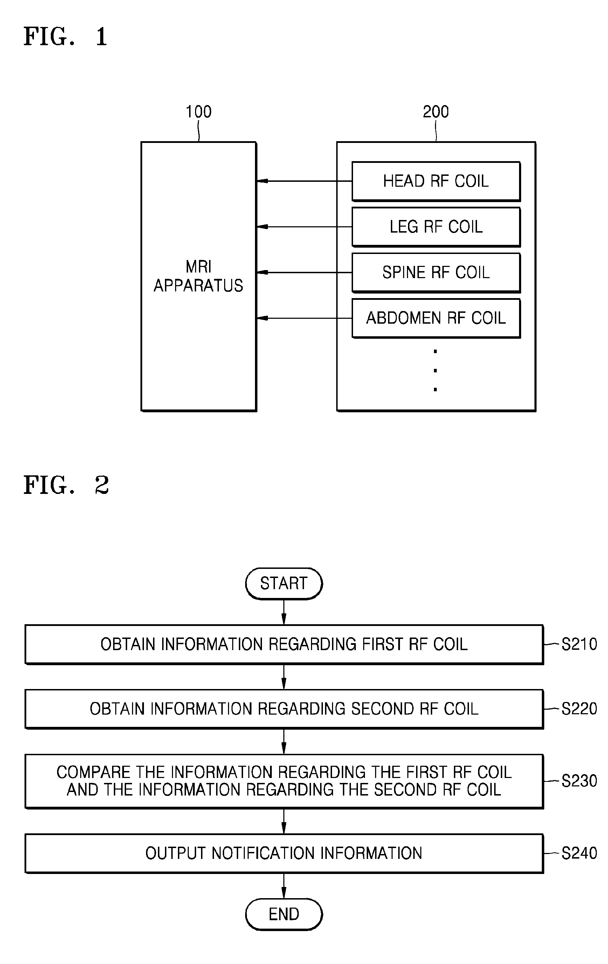

FIG. 1 is a block diagram of a magnetic resonance imaging (MRI) apparatus 100 and radio frequency (RF) coils 200 that may be connected to the MRI apparatus 100 according to an exemplary embodiment.

The various types of RF coils 200 used to photograph an object may be connected to and installed in the MRI apparatus 100.

A state in which the RF coils 200 are connected to the MRI apparatus 100 may be that the RF coils 200 and the MRI apparatus 100 are connected to each other by wire or wirelessly and transmit and receive information for photographing the object. The MRI apparatus 100 may photograph the object by using the RF coils 200 that are connected to the MRI apparatus 100 and may not photograph the object by using the RF coil 200s that are not connected to the MRI apparatus 100. The information for photographing the object may include timing information of an RF signal that is transmitted from the MRI apparatus 100 to the RF coil 200, information regarding an MR signal that is transmitted from the RF coils 200 to the MRI apparatus 100, etc.

A state in which the RF coils 200 are installed in the MRI apparatus 100 may be that the RF coils 200 are located in optional points of the MRI apparatus 100 to photograph the object.

As shown in FIG. 1, the RF coils 200 that may be connected to the MRI apparatus 100 may include a head RF coil, a leg RF coil, a spine RF coil, an abdomen RF coil, etc. The head RF coil may include different types of head RF coils according to a number of channels, size, and use.

That is, there are various types of the RF coils 200, and thus a user selects the RF coil 200 for photographing the object from among the RF coils 200 and connects the selected RF coil 200 to the MRI apparatus 100.

Therefore, the user continuously determines whether the RF coil 200 connected to the MRI apparatus 100 is an appropriate RF coil. If the object is photographed when the RF coil 200 connected to the MRI apparatus 100 is not an appropriate RF coil, there may be a problem in that the object is photographed again since an image desired by the user is not obtained.

The RF coils 200 are normally installed in the MRI apparatus 100. A state in which the RF coils 200 are normally installed in the MRI apparatus 100 may be that the RF coils 200 are located in exact points for photographing the object. For example, parts of the RF coils 200 that contact the MRI apparatus 100 are completely in contact with the MRI apparatus 100. If the object is photographed when the RF coils 200 are abnormally, i.e., improperly or incorrectly, installed in the MRI apparatus 100, there may be a problem in that quality of an image deteriorates.

When the user removes the RF coil 200 that is connected to the MRI apparatus 100 by wire from the MRI apparatus 100, the RF coils 200 may be damaged. Thus, it is important to prevent the RF coils 200 from being removed in advance when a connector of the RF coils 200 is connected to the MRI apparatus 100.

The MRI apparatus 100 according to an embodiment outputs notification information indicating that the RF coils 200 connected to the MRI apparatus 100 is to photograph the object, notification information indicating that the RF coils 200 are normally installed or the connector thereof is separated, etc., thereby increasing user convenience.

FIG. 2 is a flowchart of a notification information providing method performed by using an MRI apparatus according to an exemplary embodiment.

In operation S210, the MRI apparatus obtains information regarding a first RF coil for photographing an object.

In operation S220, the MRI apparatus obtains information regarding a second RF coil connected to the MRI apparatus.

The information regarding the first RF coil and the information regarding the second RF coil may include information regarding at least one of a part in which an RF coil is installed, a number of channels, size thereof, and use thereof.

In operation S230, the MRI apparatus compares the information regarding the first RF coil and the information regarding the second RF coil.

In operation S240, the MRI apparatus outputs notification information indicating whether the RF coil connected to the MRI apparatus corresponds to the first RF coil based on a comparison result.

A user may easily determine whether an RF coil currently connected to the MRI apparatus is necessary to photograph the object based on the notification information output by the MRI apparatus.

The MRI apparatus may output the notification information by using at least one of a speaker and a display that are attached to the second RF coil. Thus, the user may determine whether the RF coil currently connected to the MRI apparatus is an appropriate RF coil by using a gantry or the speaker or the display attached to the second RF coil immediately when the RF coil is connected to the MRI apparatus without having to separately determine whether the RF coil currently connected to the MRI apparatus is an appropriate RF coil in an operating room.

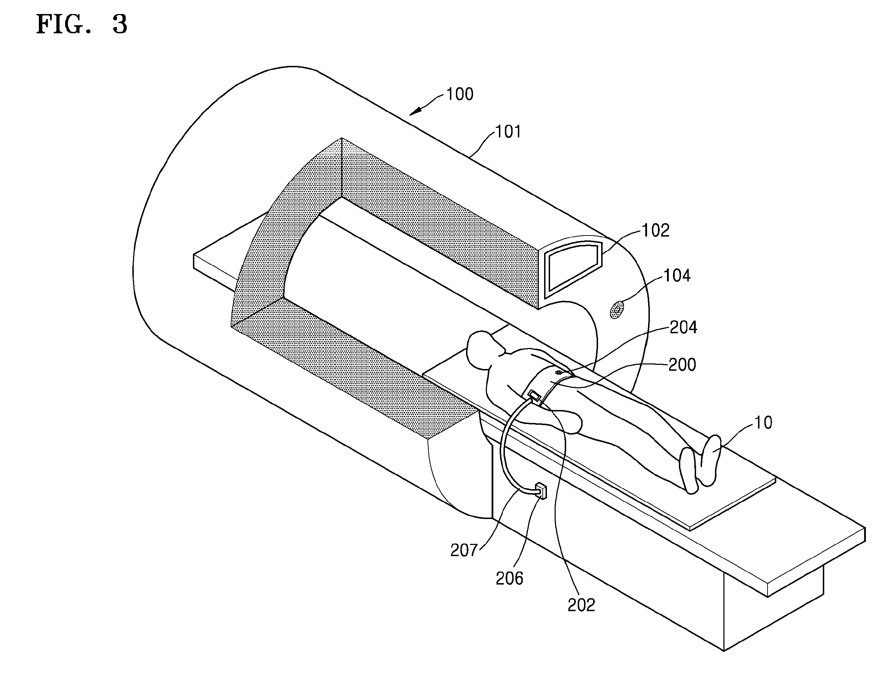

FIG. 3 is a diagram of a state in which the RF coil 200 is connected to the MRI apparatus 100. The MRI apparatus 100 may include a gantry 101 in which an object 10 is located.

Referring to FIG. 3, the RF coil 200 used to photograph an abdomen of the object 10 is connected to the MRI apparatus 100. The RF coil 200 of FIG. 3 is a wired RF coil and may be connected to the MRI apparatus 100 via a connector 206 and a connector line 207. It would be obvious to those of ordinary skill in the art that the wired RF coil may be replaced as a wireless RF coil.

As described above, speakers 104 and 204 and displays 102 and 202 may be attached to the gantry 101 and the RF coil 200. The MRI apparatus 100 may output notification information via at least one of the speakers 104 and 204 and the displays 102 and 202 that are attached to the gantry 101 or the RF coil 200.

The notification information may be output by using various methods. For example, a specific sound or screen corresponding to each of cases where the RF coil 200 that is connected to the MRI apparatus 100 is an appropriate RF coil and is not an appropriate RF coil may be output. When the RF coil 200 connected to the MRI apparatus 100 is the appropriate RF coil, no sound or screen is output, and thus a user may recognize that the RF coil 200 connected to the MRI apparatus 100 is an appropriate RF coil.

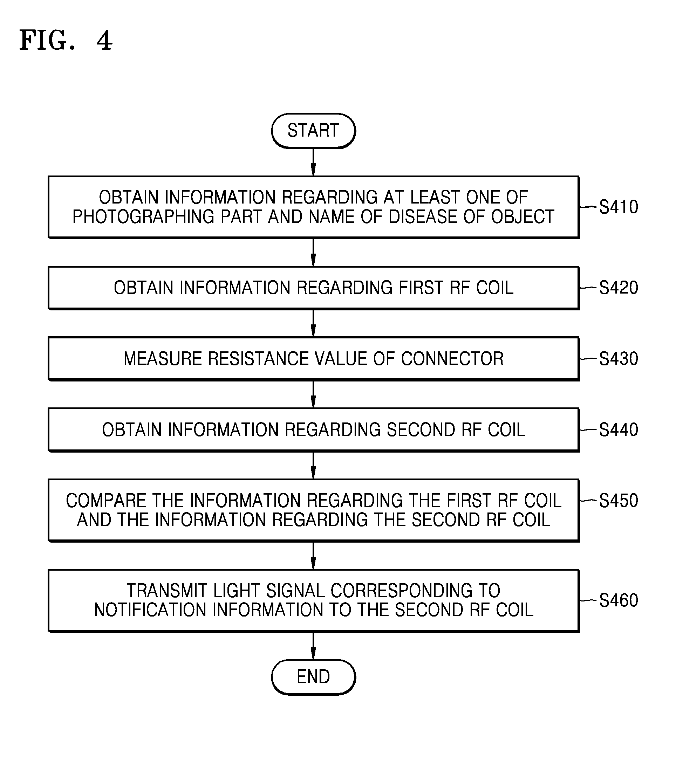

FIG. 4 is a flowchart of a notification information providing method performed by using an MRI apparatus according to another exemplary embodiment. The method of FIG. 4 is performed when a RF coil connected to the MRI apparatus is a wired RF coil.

In operation S410, the MRI apparatus obtains information regarding at least one of a photographing part of an object and a disease name. The MRI apparatus may obtain the information regarding at least one of the photographing part of the object and the disease name through a user input and may receive the information from an external server.

In operation S420, the MRI apparatus obtains information regarding a first RF coil for photographing the object based on the information regarding at least one of the photographing part of the object and the disease name.

In operation S430, the MRI apparatus measures a resistance value of a connector of a second RF coil connected to the MRI apparatus.

In operation S440, the MRI apparatus obtains information regarding the second RF coil connected to the MRI apparatus based on the measured resistance value of the connector. The measured resistance value of the connector changes according to a type of a RF coil. The MRI apparatus may match and previously store an optional RF coil and a resistance value of a connector of the corresponding RF coil, and obtain information regarding the RF coil corresponding to the resistance value of the connector of the second RF coil.

Alternatively, according to another embodiment, the MRI apparatus may directly receive the information regarding the second RF coil via the connector of the second RF coil.

In operation S450, the MRI apparatus compares the information regarding the first RF coil and the information regarding the second RF coil.

In operation S460, the MRI apparatus transmits a light signal having a color corresponding to notification information to the second RF coil.

For example, when the notification information indicates that the second RF coil corresponds to the first RF coil, the MRI apparatus may transmit a green light signal to the second RF coil, and, when the notification information indicates that the second RF coil does not correspond to the first RF coil, the MRI apparatus may transmit a red light signal to the second RF coil.

The MRI apparatus may transmit the light signal via the connector, and the second RF coil displays a color corresponding to the received light signal on a display, and thus a user may easily determine the notification information. In this regard, the display of the second RF coil may be formed of a transparent material from which the color of the light signal received from the MRI apparatus may be simply determined.

Alternatively, according to another embodiment, the MRI apparatus may transmit an electrical signal having a voltage value or a current value corresponding to the notification information to the second RF coil and output a specific sound or screen corresponding to the electrical signal received by the second RF coil.

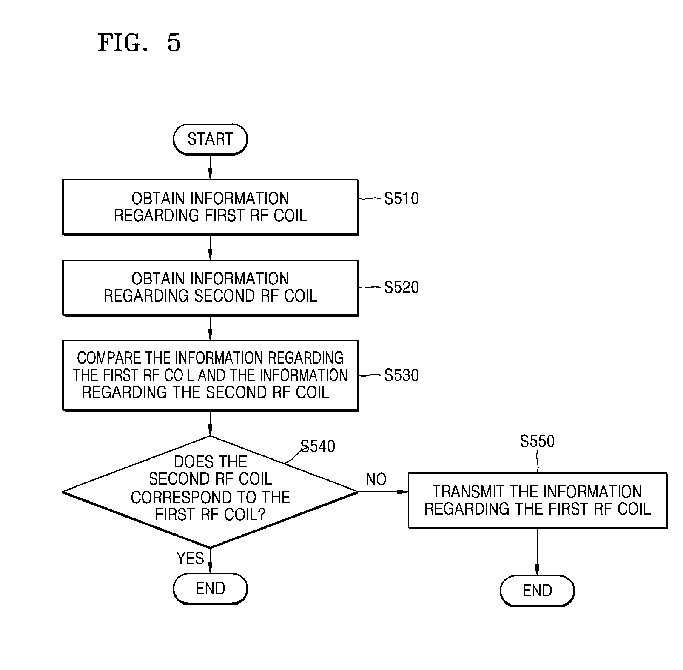

FIG. 5 is a flowchart of a notification information providing method performed by using an MRI apparatus according to another exemplary embodiment.

In operation S510, the MRI apparatus obtains information regarding a first RF coil for photographing an object. The MRI apparatus may receive the information regarding the first RF coil from an external server or obtain the information regarding the first RF coil based on information regarding a disease name of the object or a photographing part thereof.

In operation S520, the MRI apparatus obtains information regarding a second RF coil connected to the MRI apparatus. When the second RF coil is a wired RF coil, the MRI apparatus may obtain the information regarding the second RF coil by measuring a resistance value of a connector of the second RF coil and via the connector of the second RF coil. Alternatively, when the second RF coil is a wireless RF coil connected to the MRI apparatus based on a first wireless communication method, the MRI apparatus may receive the information regarding the second RF coil from the second RF coil by using the first wireless communication method.

In operation S530, the MRI apparatus compares the information regarding the first RF coil and the information regarding the second RF coil.

In operation S540, the MRI apparatus determines whether the second RF coil corresponds to the first RF coil based on a result of comparison obtained in operation S530.

When the second RF coil corresponds to the first RF coil, the MRI apparatus outputs notification information indicating whether the second RF coil is an appropriate RF coil or outputs no notification information, and thus a user may recognize that the RF coil is the appropriate RF coil.

In operation S550, when the second RF coil does not correspond to the first RF coil, the MRI apparatus transmits the information regarding the first RF coil to one or more RF coils that are not connected to the MRI apparatus. The MRI apparatus may transmit the information regarding the first RF coil by using a second wireless communication method. The second wireless communication method may include various types of short or long distance wireless communication methods such as WiFi, Bluetooth, and RFID.

Each of one or more RF coils determines whether information regarding each RF coil and the information regarding the first RF coil correspond to each other. A RF coil corresponding to the information regarding the first RF coil from among the one or more RF coils may output sound and light and notify the user of the RF coil that corresponds to the information regarding the first RF coil. The user may easily determine the RF coil that outputs sound and light as a RF coil for photographing the object.

Meanwhile, although the MRI apparatus may determine whether the second RF coil connected to the MRI apparatus corresponds to the first RF coil above, the second RF coil may also determine whether the second RF coil corresponds to the first RF coil.

For example, the second RF coil may receive the information regarding the first RF coil from the MRI apparatus, compare the information regarding the first RF coil and the information regarding the second RF coil, determine whether the second RF coil corresponds to the first RF coil, and output the notification information based on a result of the determination.

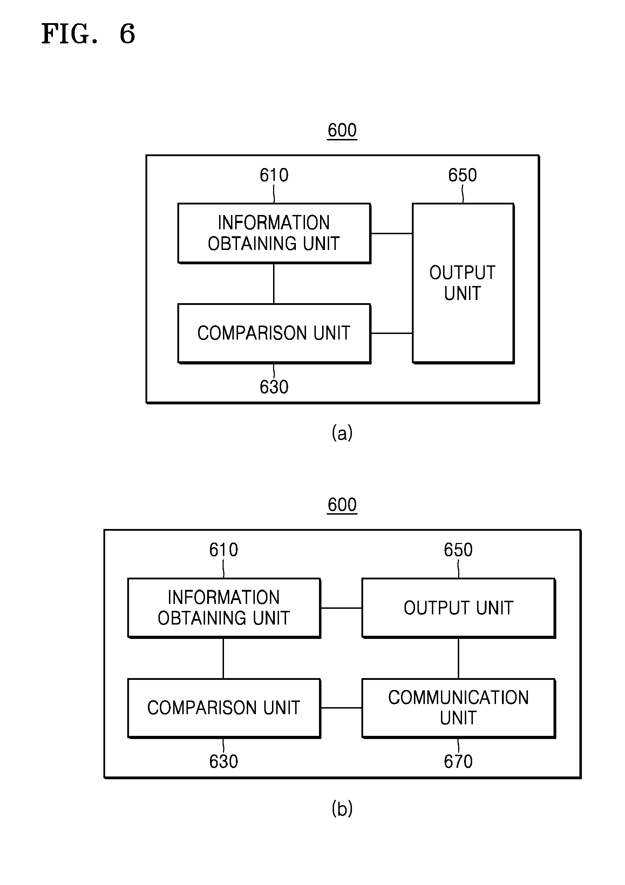

FIG. 6A is a block diagram illustrating a configuration of an MRI apparatus 600 according to an exemplary embodiment. FIG. 6B is a block diagram illustrating a configuration of the MRI apparatus 600 according to another exemplary embodiment.

Referring to FIG. 6A, the MRI apparatus 600 according to an exemplary embodiment may include an information obtaining unit 610, e.g., information obtainer, a comparison unit 630, e.g., a comparer, and an output unit 650, e.g., outputter. The information obtaining unit 610 and the comparison unit 630 may be configured as microprocessors.

The information obtaining unit 610 obtains information regarding a first RF coil for photographing an object and information regarding a second RF coil connected to the MRI apparatus 600.

The information obtaining unit 610 may obtain the information regarding the first RF coil based on information regarding at least one of a disease name of the object and a photographing part thereof and receive the information regarding the first RF coil from an external server.

The information obtaining unit 610 may also obtain the information regarding the second RF coil by measuring a resistance value of a connector of the second RF coil connected to the MRI apparatus 600 and receive the information regarding the second RF coil from the second RF coil by wire or wirelessly.

The comparison unit 630 compares the information regarding the first RF coil and the information regarding the second RF coil. The information regarding the first RF coil and the information regarding the second RF coil may include information regarding at least one of a part in which an RF coil is installed, a number of channels, size thereof, and use thereof.

The output unit 650 outputs notification information indicating whether the second RF coil corresponds to the first RF coil based on a result of the comparison.

The output unit 650 may output the notification information via at least one of a speaker and a display that are attached to a gantry of the MRI apparatus 600 or the second RF coil. Alternatively, when the second RF coil is a wired RF coil, the output unit 650 may transmit a light signal having a color corresponding to the notification information to the second RF coil and display the color on the display attached to the second RF coil.

Referring to FIG. 6B, the MRI apparatus 600 according to another exemplary embodiment may include the information obtaining unit 610, the comparison unit 630, the output unit 650, and a communication unit 670, e.g., a communicator. The information obtaining unit 610, the comparison unit 630, and the output unit 650 are described with reference to FIG. 6A, and thus detailed descriptions thereof are omitted here.

When the second RF coil connected to the MRI apparatus 600 does not correspond to the first RF coil by using a first wired or wireless communication method, the communication unit 670 may transmit the information regarding the first RF coil to one or more RF coils that are not connected to the MRI apparatus 600 based on a second wireless communication method. Each of one or more RF coils determines whether information regarding each RF coil and the information regarding the first RF coil correspond to each other. A RF coil corresponding to the information regarding the first RF coil among the one or more RF coils may output sound and light and notify the user of the RF coil that corresponds to the information regarding the first RF coil. The user may easily determine the RF coil that outputs sound and light as a RF coil for photographing the object.

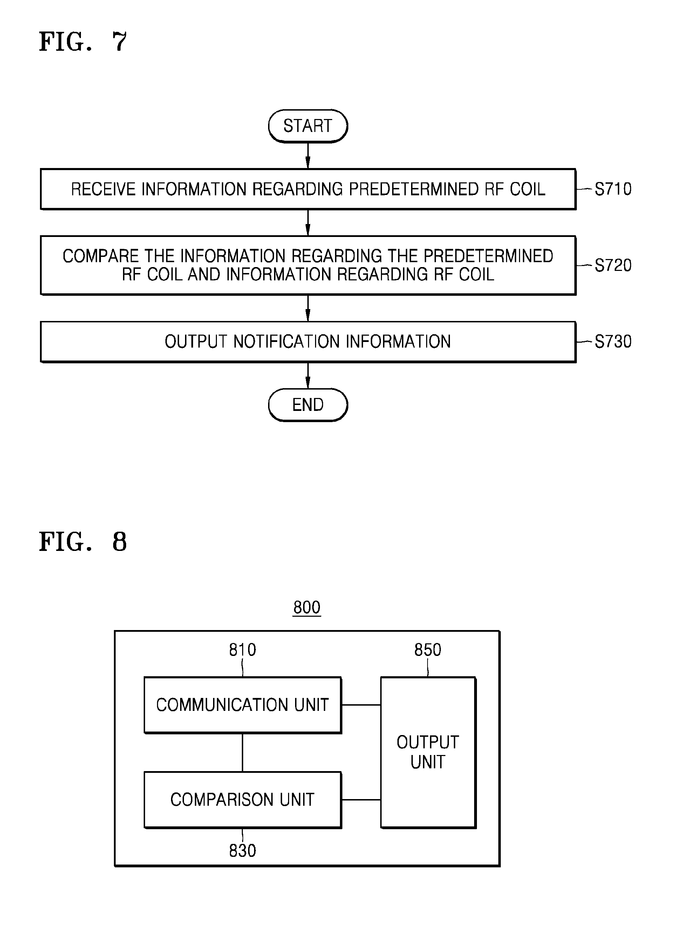

FIG. 7 is a flowchart of a notification information providing method performed by using a RF coil according to an exemplary embodiment. The RF coil used to perform the method of FIG. 7 is not connected to an MRI apparatus.

In operation S710, the RF coil receives information regarding a predetermined RF coil for photographing an object from the MRI apparatus.

In operation S720, the RF coil compares the received information regarding the predetermined RF coil and information regarding the RF coil.

In operation S730, when the RF coil corresponds to the predetermined RF coil, the RF coil outputs notification information indicating that the RF coil corresponds to the predetermined RF coil.

That is, the RF coil according to an exemplary embodiment may output the notification information such as sound and light indicating that the RF coil corresponds to the predetermined RF coil for photographing the object and notify a user of the RF coil that corresponds to the predetermined RF coil when the RF coil is not connected to the MRI apparatus. The user may easily determine the RF coil that outputs sound and light as a RF coil for photographing the object.

FIG. 8 is a block diagram illustrating a configuration of a RF coil 800 according to an exemplary embodiment.

The RF coil 800 according to an exemplary embodiment may include a communication unit 810, a comparison unit 830, and an output unit 850. The RF coil 800 is not connected to an MRI apparatus to photograph an object.

The communication unit 810 may receive information regarding a predetermined RF coil for photographing the object from the MRI apparatus. The RF coil 800 is not connected to the MRI apparatus, and thus the communication unit 810 may receive the information regarding the predetermined RF coil based on a second wireless communication method other than a first wireless communication method of connecting the RF coil 800 to the MRI apparatus.

The comparison unit 830 may compare the information regarding the predetermined RF coil and information regarding the RF coil 800.

When the RF coil 800 corresponds to the predetermined RF coil, the output unit 850 may output notification information indicating that the RF coil 800 corresponds to the predetermined RF coil.

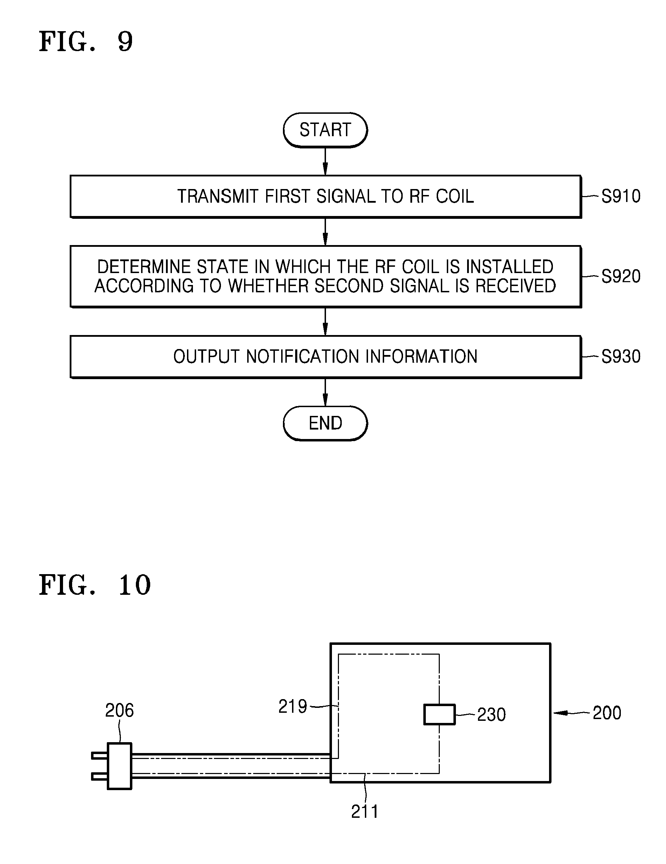

FIG. 9 is a flowchart of a notification information providing method performed by using an MRI apparatus according to another exemplary embodiment.

In operation S910, the MRI apparatus transmits a first signal to a RF coil connected to the MRI apparatus via a connector by wire. For example, the MRI apparatus may transmit the first signal through a first signal connection path connected to the RF coil.

In operation S920, the MRI apparatus determines a state in which the RF coil is installed according to whether a second signal corresponding to the first signal is received from the RF coil. For example, the MRI apparatus may determine whether the RF coil is normally installed in the MRI apparatus according to whether the second signal is received through a second signal connection path connected to the RF coil. The first signal and the second signal may be the same and may include light signals or electrical signals.

In more detail, the MRI apparatus may determine that the RF coil is normally installed in the MRI apparatus when the second signal is received, and may determine that the RF coil is abnormally installed in the MRI apparatus when the second signal is not received. To the contrary, when the second signal is received, the MRI apparatus may determine that the RF coil is abnormally installed in the MRI apparatus, and, when the second signal is not received, may determine that the RF is normally installed in the MRI apparatus.

In operation S930, when the MRI apparatus determines that the RF coil is abnormally installed in the MRI apparatus, the MRI apparatus outputs notification information as guidance indicating that a connector is to be separated from the MRI apparatus or that the RF coil is to be normally installed in the MRI apparatus.

The MRI apparatus may output alarm sound or alarm screen as the notification information. The user may be informed that the RF coil is abnormally installed in the MRI apparatus in a state where a connector of the RF coil is not separated from the MRI apparatus through the alarm sound or alarm screen.

The RF coil being abnormally installed in the MRI apparatus may mean that the RF coil is removed from the MRI apparatus. That is, the RF coil is abnormally installed in the MRI apparatus means that the RF coil that needs to contact the MRI apparatus does wholly or partly not contact the MRI apparatus, and thus the RF coil being abnormally installed in the MRI apparatus may mean that the user has moved the MRI apparatus to remove the RF coil.

Therefore, when the MRI apparatus outputs the notification information, the user may separate the connector of the RF coil from the MRI apparatus and remove the RF coil or may recognize that the RF coil is normally installed in the MRI apparatus.

The MRI apparatus according to an exemplary embodiment may transmit the first signal to the RF coil and set time for determining a state in which the RF coil is installed in various ways.

For example, the MRI apparatus may transmit the first signal to the RF coil and determine the state in which the RF coil is installed when preparing photographing of the object.

The MRI apparatus determines whether the MRI apparatus photographs the object and does not transmit the first signal while the object is being photographed, and thus the MRI apparatus may not determine the state in which the RF coil is installed since flow of the first signal and the second signal may affect the MRI photographing.

A state in which the object is photographed may include a state in which a magnetostatic field or a gradient magnetic field is formed in a gantry of the MRI apparatus or a state in which an RF signal is irradiated on the object.

The MRI apparatus may not determine the state in which the RF coil is installed within a predetermined period of time after the object is completely photographed and may output an alarm sound or alarm screen only if the RF coil is connected to the MRI apparatus via the connector and guide the user to separate the connector of the RF coil from the MRI apparatus so as to prevent the user from moving or removing the RF coil in advance in a state in which the connector is connected to the MRI apparatus.

FIG. 10 is an exemplary diagram of a RF coil 200 that is to be installed in an MRI apparatus according to an exemplary embodiment.

Referring to FIG. 10, the RF coil 200 may include the connector 206 connected to the MRI apparatus, a first signal connection path 211, a second signal connection path 219, and a switch 230.

The first signal connection path 211 is used as a flow path by a first signal output from the MRI apparatus. The second signal connection path 219 is used as a flow path by a second signal to the MRI apparatus.

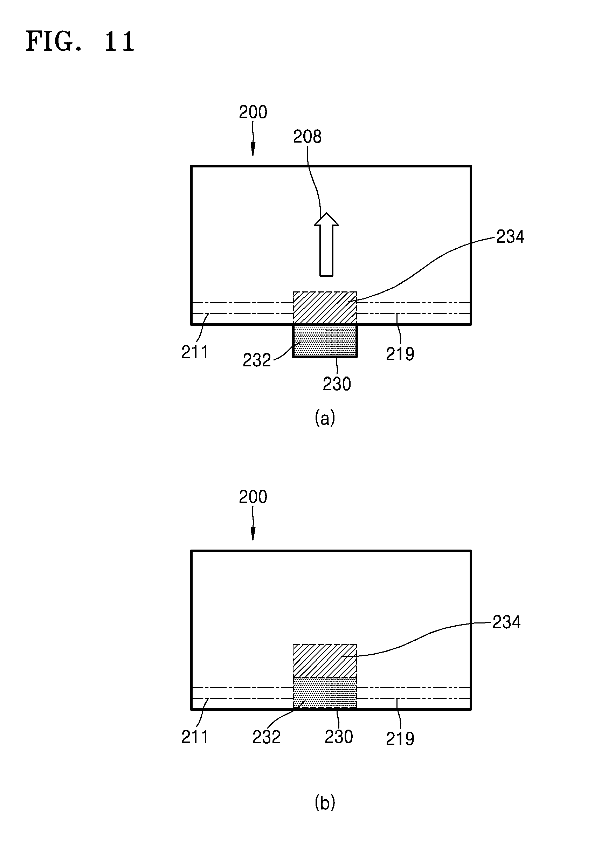

The switch 230 may contact the MRI apparatus and may be disposed between the first signal connection path 211 and the second signal connection path 219. The switch 230 that contacts the MRI apparatus may move in a first direction, which will be described with reference to FIGS. 11A and 11B.

FIG. 11A is a lateral view of the RF coil 200 of FIG. 10. FIG. 11B is a diagram of the switch 230 of FIG. 11A that moves in a first direction 208.

As described above, the switch 230 may move in the first direction 208 since the switch 230 contacts an MRI apparatus. The switch 230 may include a signal blocking unit 234, e.g, a signal blocker, and a signal penetrating unit 232, e.g., a signal penetrator, that may be disposed in a bottom portion of the signal blocking unit 234. That is, if the switch 230 does not contact the MRI apparatus, the signal blocking unit 234 of the switch 230 is disposed between the first signal connection path 211 and the second signal connection path 219, and thus the first signal connection path 211 and the second signal connection path 219 are not connected to each other. Accordingly, a first signal that flows through the first signal connection path 211 does not enter the second signal connection path 219, and thus the MRI apparatus does not receive a second signal that flows through the second signal connection path 219. In other words, when the switch 230 of the RF coil 200 that needs to contact the MRI apparatus does not contact the MRI apparatus, the MRI apparatus may not receive the second signal that flows along the second signal connection path 219, and thus the MRI apparatus may be informed that the RF coil 200 is abnormally installed.

To the contrary, the switch 230 may move in the first direction 208 since the switch 230 contacts the MRI apparatus. Referring to FIG. 11B, when the switch 230 moves in the first direction 208, the signal penetrating unit 232 of the switch 230 is disposed between the first signal connection path 211 and the second signal connection path 219, and thus the first signal that flows along the first signal connection path 211 enters the second signal connection path 219. When the MRI apparatus receives the second signal via the second signal connection path 219, the MRI apparatus may determine that the RF coil 200 is normally installed.

Although the signal penetrating unit 232 of the switch 230 is disposed in a bottom portion of the signal blocking unit 234 in FIGS. 11A and 11B, the signal penetrating unit 232 may be disposed in a top portion of the signal blocking unit 234. In this case, the MRI apparatus may determine that the RF coil 200 is normally installed when the MRI apparatus does not receive the second signal via the second signal connection path 219, and the MRI apparatus may determine that the RF coil 200 is abnormally installed when the MRI apparatus receives the second signal via the second signal connection path 219.

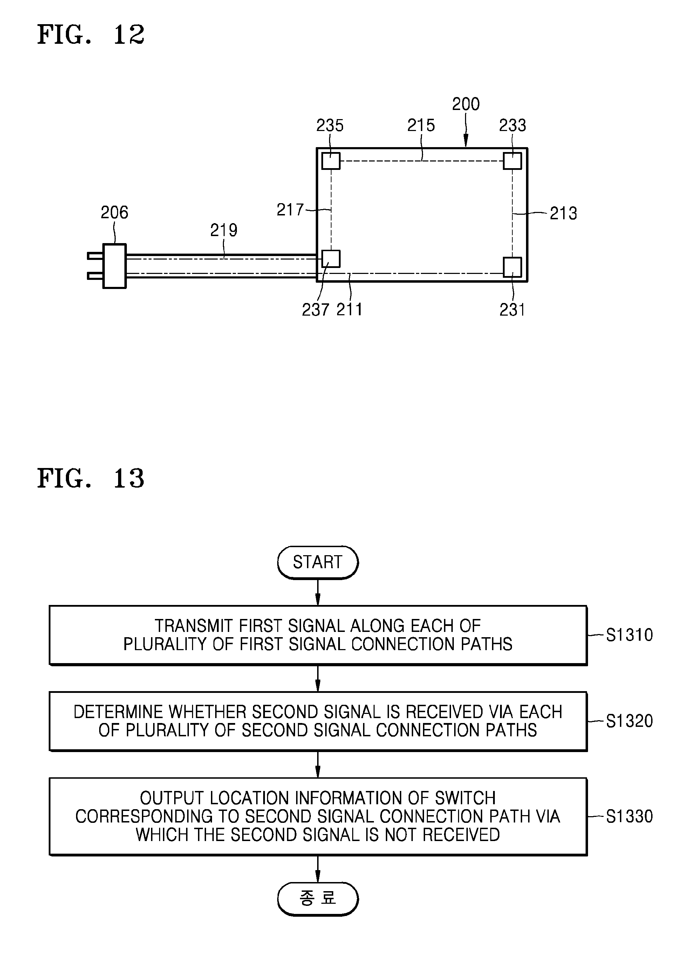

Although one switch 230 is included in the RF coil 200 in FIGS. 10 through 11B, a plurality of switches may be included in the RF coil 200. This will be described with reference to FIG. 12.

FIG. 12 is an exemplary diagram of the RF coil 200 that is to be installed in an MRI apparatus according to another exemplary embodiment.

The RF coil 200 of FIG. 12 includes four switches 231, 233, 235, and 237. The first switch 231 is connected to the first signal connection path 211. The fourth switch 234 is connected to the second signal connection path 219. A third signal connection path 213 may be disposed between the first switch 231 and the second switch 233. A fourth signal connection path 215 may be disposed between the second switch 233 and the third switch 235. A fifth signal connection path 217 may be disposed between the third switch 233 and the fourth switch 237.

As described above, the first through fourth switches 231, 233, 235, and 237 may move in the first direction 208 as they contact the MRI apparatus.

When the first through fourth switches 231, 233, 235, and 237 contact the MRI apparatus and move in the first direction 208, the first through fifth signal connection paths 211, 213, 215, 217, and 219 may be connected to each other, and thus a first signal flowing along the first signal connection path 211 may enter the second signal connection path 219.

The MRI apparatus may determine whether a second signal is received via the second signal connection path 219 and determine whether the RF coil 200 precisely contacts the MRI apparatus, in other words, whether the first through fourth switches 231, 233, 235, and 237 that are included in the RF coil 200 move in the first direction 208.

FIG. 13 is a flowchart of a notification information providing method performed by using an MRI apparatus according to another exemplary embodiment. FIG. 13 explains a method of providing location information of a part of a RF coil that is abnormally installed in the MRI apparatus.

In operation S1310, the MRI apparatus transmits a first signal along each of a plurality of first signal connection paths connected to the RF coil.

In operation S1320, the MRI apparatus determines whether a second signal is received from the RF coil via each of a plurality of second signal connection paths connected to the RF coil.

In operation S1330, the MRI apparatus outputs location information of a switch corresponding to a second signal connection path via which the second signal is not received from among the plurality of second signal connection paths.

A user may determine a part of the RF coil that does not contact the MRI apparatus from the location information of the switch output by the MRI apparatus.

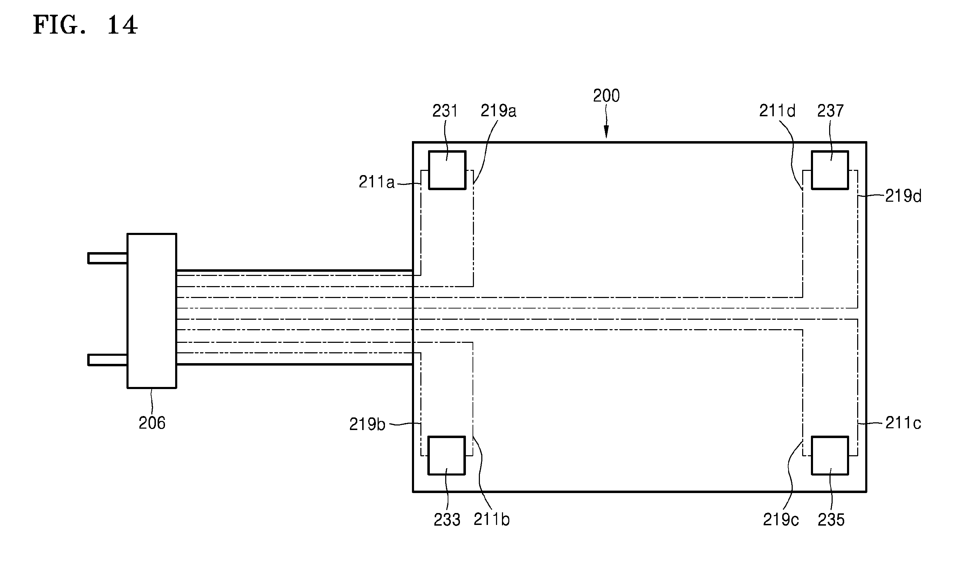

The RF coil including the plurality of first signal connection paths and the plurality of second signal connection paths is shown in FIG. 14.

FIG. 14 is an exemplary diagram of the RF coil 200 that is to be installed in an MRI apparatus according to another exemplary embodiment.

The RF coil 200 of FIG. 14 may include the four switches 231, 233, 235, and 237, four first signal connection paths 211a, 211b, 211c, and 211d, and four second signal connection paths 219a, 219b, 219c, and 219d that are respectively connected to the four switches 231, 233, 235, and 237.

The first through fourth switches 231, 233, 235, and 237 may be located in a part of the RF coil 200 that is to contact the MRI apparatus and may move in a first direction since the RF coil 200 contacts the MRI apparatus. When the RF coil 200 contacts the MRI apparatus and thus the first through fourth switches 231, 233, 235, and 237 move in the first direction, the four first signal connection paths 211a, 211b, 211c, and 211d and the four second signal connection paths 219a, 219b, 219c, and 219d that respectively correspond to the four switches 231, 233, 235, and 237 may be connected to each other.

The MRI apparatus may transmit a first signal along each of the first signal connection paths 211a, 211b, 211c, and 211d and determine whether a second signal is received via each of the our second signal connection paths 219a, 219b, 219c, and 219d. If the second signal is not received from the second signal connection path 219b corresponding to the second switch 233, since the second switch 233 does not contact the MRI apparatus, the MRI apparatus outputs location information of the second switch 233, and thus a user may recognize that the RF coil 200 is abnormally installed in the MRI apparatus at a location of the second switch 233.

Although the four first signal connection paths 211a, 211b, 211c, and 211d, and the four second signal connection paths 219a, 219b, 219c, and 219d are included in a connector line in FIGS. 10, 12, and 14, it will be obvious to those of ordinary skill in the art that the four first signal connection paths 211a, 211b, 211c, and 211d, and the four second signal connection paths 219a, 219b, 219c, and 219d may be included in a line other than the connector line.

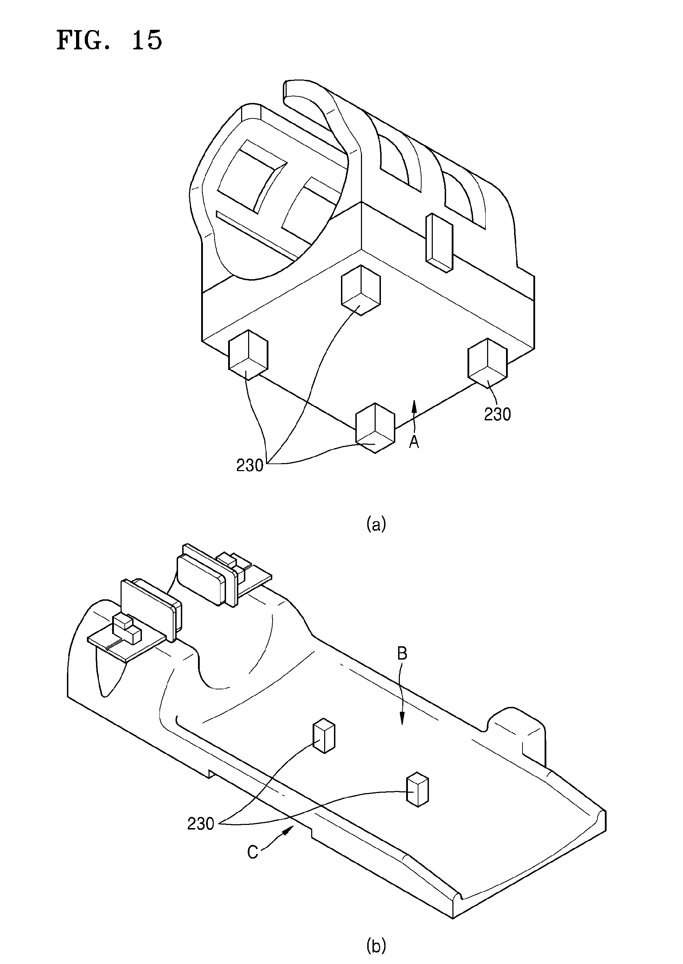

FIG. 15A is a diagram of a head RF coil including the switch 230. FIG. 15B is a diagram of a spine RF coil including the switch 230.

The switch 230 included in a RF coil according to an exemplary embodiment may be located in a surface of the RF coil that contacts an MRI apparatus or an object.

A bottom surface A of the head RF coil of FIG. 15A contacts the MRI apparatus, and thus the switch 230 may be located in the bottom surface A of the head RF coil.

A bottom surface C of the spine RF coil of FIG. 15B contacts the MRI apparatus, and a top surface B thereof contacts the object, and thus the switch 230 may be located in the bottom surface C or the top surface B of the head RF coil. The switch 230 is located in the top surface B of the head RF coil in FIG. 15B.

The switch 230 may be located in a surface of a RF coil that contacts the object or the MRI apparatus, other than the head RF coil and the spine RF coil.

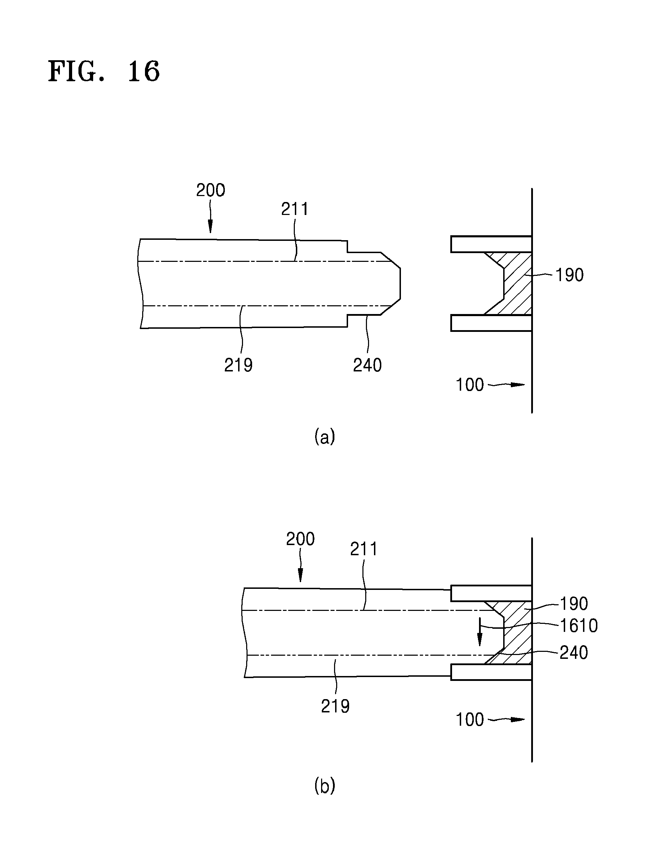

FIG. 16A is an exemplary diagram of the RF coil 200 that is to be installed in the MRI apparatus 100 according to another exemplary embodiment. FIG. 16B is a diagram of a signal reflecting unit 190 and a contact unit 240 of FIG. 16A that contact each other.

The RF coil 200 of FIG. 16A may include the first signal connection path 211, the second signal connection path 219, and the contact unit 240. The MRI apparatus 100 of FIG. 16A may include the signal reflecting unit 190 that reflects a first signal.

The first signal connection path 211 is connected to the MRI apparatus 100 in such a manner that the first signal output from the MRI apparatus 100 flows along the first signal connection path 211. The second signal connection path 219 is connected to the MRI apparatus 100 in such a manner that a second signal output from the MRI apparatus 100 flows through the second signal connection path 219. The first signal connection path 211 and the second signal connection path 219 may not be connected to each other and may extend to the contact unit 240.

Referring to FIG. 16A, the first signal connection path 211 and the second signal connection path 219 are not connected to each other, and thus the first signal output from the MRI apparatus 100 does not enter the second signal connection path 219, whereby the MRI apparatus 100 does not receive the second signal via the second signal connection path 219.

Referring to FIG. 16B, when the contact unit 240 of the RF coil 200 contacts the signal reflecting unit 190, the first signal flowing through the first signal connection path 211 is reflected by the signal reflection unit 190, e.g., a signal reflector, and a reflected signal 1310 enters the second signal connection path 219, whereby the MRI apparatus 100 receives the second signal via the second signal connection path 219.

When the first signal is reflected by the signal reflecting unit 190 and the second signal is received via the second signal connection path 219, the MRI apparatus 100 may determine that the RF coil 200 is normally installed in the MRI apparatus 100.

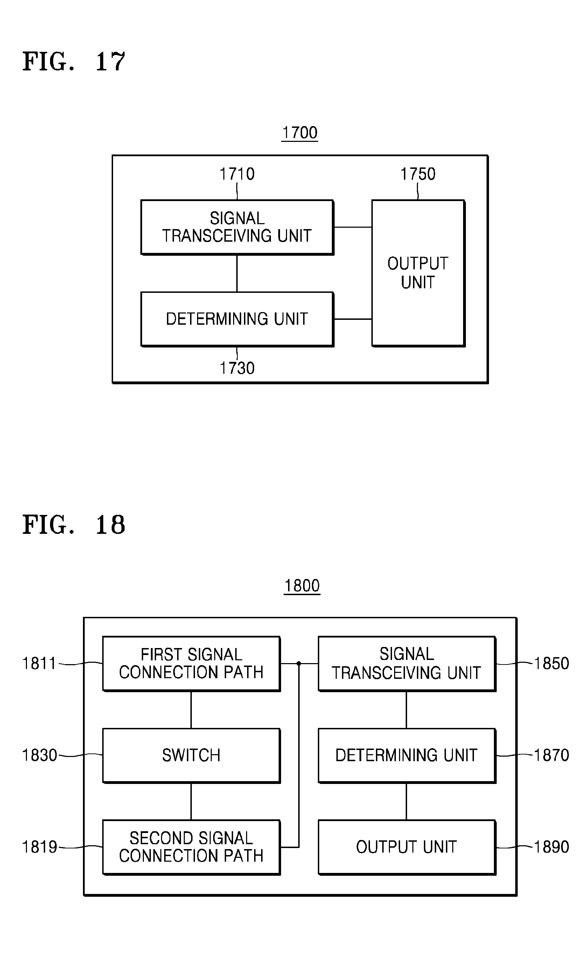

FIG. 17 is a block diagram illustrating a configuration of an MRI apparatus 1700 according to another exemplary embodiment.

Referring to FIG. 17, the MRI apparatus 1700 according to another exemplary embodiment may include a signal transceiving unit 1710, e.g., a signal transceiver, a determining unit 1730, e.g., a determiner, and an output unit 1750, e.g., an outputter. The determining unit 1730 may be configured as a microprocessor.

The signal transceiving unit 1710 transmits a first signal to an RF coil connected to the MRI apparatus 1700 via a connector by wire. The signal transceiving unit 1710 may receive a second signal from the RF coil. For example, the signal transceiving unit 1710 may transmit the first signal along a first signal connection path connected to the RF coil and may receive the second signal via a second signal connection path. The first signal and the second signal may be the same and may include light signals or electrical signals.

The signal transceiving unit 1710 may transmit the first signal along each of a plurality of first signal connection paths connected to the RF coil, and may receive the second signal via each of a plurality of second signal connection paths connected to the RF coil.

The determining unit 1730 determines a state in which the RF coil is installed according to whether the signal transceiving unit 1710 receives the second signal corresponding to the first signal from the RF coil.

When the RF coil is abnormally installed in the MRI apparatus 1700, the output unit 1750 may guide the connector to be separated from the MRI apparatus 1700 or output notification information as guidance indicating that the RF coil is to be normally installed in the MRI apparatus 1700.

The output unit 1750 may output the notification information through at least one of a speaker and a display that are connected to a gantry of the MRI apparatus 1700 or the RF coil.

The MRI apparatus 1700 according to an exemplary embodiment may a set time for transmitting the first signal to the RF coil and determining a state in which the RF coil is installed in various ways.

For example, the MRI apparatus 1700 may determine the state in which the RF coil is installed when preparing photographing of an object.

The MRI apparatus 1700 determines whether the MRI apparatus 1700 photographs the object and does not transmit the first signal while the object is being photographed, and thus the MRI apparatus 1700 may not determine the state in which the RF coil is installed since flow of the first signal and the second signal may affect MRI photographing.

The MRI apparatus 1700 may not determine the state in which the RF coil is installed within a predetermined period of time after the object is completely photographed and may output an alarm sound or alarm screen only if the RF coil is connected to the MRI apparatus 1700 via the connector and guide the user to separate the connector of the RF coil from the MRI apparatus 1700 so as to prevent the user from moving or removing the RF coil in advance in a state in which the connector is connected to the MRI apparatus 1700.

FIG. 18 is a block diagram illustrating a configuration of a RF coil 1800 according to another exemplary embodiment.

Referring to FIG. 18, the RF coil 1800 according to another exemplary embodiment may include a first signal connection path 1811, a second signal connection path 1819, a switch 1830, a signal transceiving unit 1850, e.g., a signal transceiver, a determining unit 1870, e.g., a determiner, and an output unit 1890, e.g., an outputter.

Although a first signal flowing along a first signal connection path is output from an MRI apparatus, and a second signal flowing along a second signal connection path is output from the MRI apparatus in FIGS. 10, 11A, 11B, 12, 14, 15A, and 15B, the RF coil 1800 according to another exemplary embodiment may include the signal transceiving unit 1850 that transmits the first signal along the first signal connection path 1811 and receives the second signal via the second signal connection path 1819.

For example, when the RF coil 1800 connected to the MRI apparatus is a wireless RF coil, the wireless RF coil does not receive the first signal from the MRI apparatus, the signal transceiving unit 1850 of the wireless RF coil transmits the first signal along the first signal connection path 1811 and receives the second signal via the second signal connection path 1819.

The switch 1830 may move in a first direction since the RF coil 1800 contacts the MRI apparatus or an object, so that the first signal connection path 1811 and the second signal connection path 1819 may be connected to each other, and the second signal corresponding to the first signal flowing along the first signal connection path 1811 may flow along the second signal connection path 1819.

When the RF coil 1800 is abnormally installed in the MRI apparatus, the output unit 1890 outputs notification information as guidance indicating that the RF coil 1800 is to be normally installed in the MRI apparatus. When the RF coil 1800 is a wired RF coil, the output unit 1890 may output notification information as guidance indicating that the RF coil 1800 is to be normally installed in the MRI apparatus or that a connector of the wired RF coil is to be separated from the MRI apparatus.

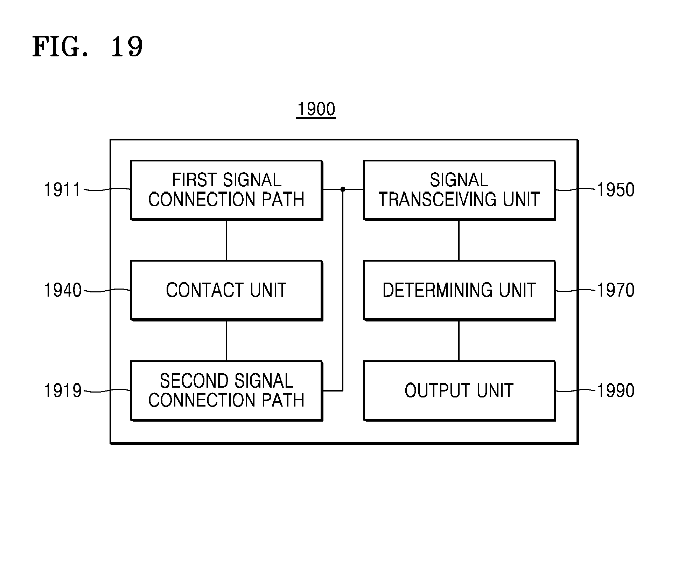

FIG. 19 is a block diagram illustrating a configuration of a RF coil 1900 according to another exemplary embodiment.

Referring to FIG. 19, the RF coil 1900 according to another exemplary embodiment may include a first signal connection path 1911, a second signal connection path 1919, a contact unit 1940, e.g., a contact, a signal transceiving unit 1950, e.g., a signal transceiver, a determining unit 1970, e.g., a determiner, and an output unit 1990, e.g., an outputter.

Although a first signal flowing along a first signal connection path is output from an MRI apparatus, and a second signal flowing along a second signal connection path is output from the MRI apparatus in FIG. 16, the RF coil 1900 according to another exemplary embodiment may include the signal transceiving unit 1950 that transmits the first signal along the first signal connection path 1911 and receives the second signal via the second signal connection path 1919.

The first signal connection path 1911 and the second signal connection path 1919 may not be connected to each other and may extend to the contact unit 1940.

The contact unit 1940 may contact a signal reflecting unit of an MRI apparatus. In this case, the first signal flowing along the first signal connection path 1911 may be reflected by the signal reflecting unit of the MRI apparatus and the second signal corresponding to the first signal may flow along the second signal connection path 1919.

The determining 1970 determines a state in which the RF coil 1900 is installed according to whether the signal transceiving unit 1950 receives the second signal via the second signal connection path 1919.

When the RF coil 1900 is abnormally installed in the MRI apparatus, the output unit 1990 outputs notification information as guidance indicating that the RF coil 1900 is to be normally installed in the MRI apparatus. When the RF coil 1900 is a wired RF coil, the output unit 1990 may output notification information as guidance indicating that the RF coil 1900 is to be normally installed in the MRI apparatus or that a connector of the wired RF coil is to be separated from the MRI apparatus.

The RF coils 1800 and 1900 of FIGS. 18 and 19, respectively, transmit the first signal, and thus a time for determining states in which the RF coils 1800 and 1900 are installed may be determined in various ways.

For example, the RF coils 1800 and 1900 transmit the first signal when preparing photographing of an object so that the RF coils 1800 and 1900 may determine the states in which the RF coils 1800 and 1900 are installed in the MRI apparatus.

The RF coils 1800 and 1900 determine whether the MRI apparatus photographs the object and do not transmit the first signal while the object is being photographed, and thus the RF coils 1800 and 1900 may not determine the states in which the RF coils 1800 and 1900 are installed in the MRI apparatus since flow of the first signal and the second signal may affect the MRI photographing.

The RF coils 1800 and 1900 may not determine the states in which the RF coils 1800 and 1900 are installed in the MRI apparatus within a predetermined period of time after the object is completely photographed and may output an alarm sound or alarm screen only if the RF coils 1800 and 1900 are connected to the MRI apparatus and guide a user to separate connectors of the RF coils 1800 and 1900 from the MRI apparatus so as to prevent the user from moving or removing the RF coils 1800 and 1900 in advance in a state in which the connectors are connected to the MRI apparatus.

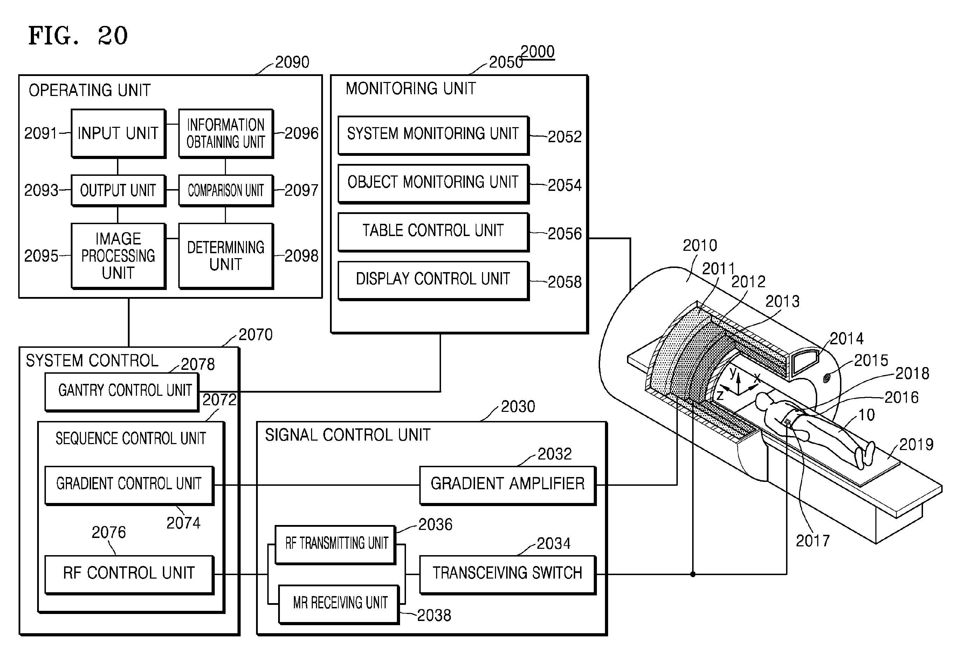

FIG. 20 is a block diagram illustrating a configuration of an MRI apparatus 2000 according to another exemplary embodiment.

Referring to FIG. 20, the MRI apparatus 2000 may include a gantry 2010, a signal control unit 2030, e.g., a signal controller, a monitoring unit 2050, e.g., a monitor, a system control unit 2070, e.g., a system controller, and an operating unit 2090, e.g., an operator.

The gantry 2010 blocks electronic waves generated by a main magnet 2011, a gradient coil 2012, a fixed RF coil 2013, and a removable RF coil 2016 from being radiated on the outside. A magnetostatic field or a gradient magnetic field is formed in a bore of the gantry 2010 so that an RF signal is irradiated on an object 10.

The main magnet 2011, the gradient coil 2012, and the fixed RF coil 2013 may be disposed in a predetermined direction of the gantry 2010. The predetermined direction may include a coaxial cylindrical direction. The object 10 may be placed on a table 2019 that may be inserted into a cylinder along a cylindrical horizontal axis.

The main magnet 2011 generates a magnetostatic field or a static magnetic field used to align directions of magnetic dipole moments of nuclei included in the object 10 in a uniform direction. The stronger and more uniform the magnetic field generated by the main magnet 2011, the relatively more precise and accurate an MR image of the object 10 may be obtained.

The gradient coil 2012 includes X, Y, and Z coils that generate a gradient magnetic field in X, Y, and Z axial directions that are perpendicular to each other. The gradient coil 2012 may induce different resonance frequencies for parts of the object 10 and provide location information of each part of the object 10.

The fixed RF coil 2013 and the removable RF coil 2016 may radiate the RF signal to a patient and receive the MR signal emitted from the patient. In more detail, the fixed RF coil 2013 and the removable RF coil 2016 may transmit the RF signal having the same frequency as that of a precession to the patient toward a nucleus that performs the precession and then stops transmitting the RF signal, and may receive the MR signal emitted from the patient.

For example, the fixed RF coil 2013 and the removable RF coil 2016 may generate an electronic wave signal having a RF corresponding to a type of a nucleus, for example, the RF signal, and apply the electronic wave signal to the object 10 so as to transit the nucleus from a low energy state to a high energy state. If the electronic wave signal generated by the fixed RF coil 2013 and the removable RF coil 2016 is applied to a nucleus, the nucleus may be transited from the nucleus from a low energy state to a high energy state. Thereafter, if the electronic wave signal generated by the fixed RF coil 2013 and the removable RF coil 2016 disappears, the nucleus to which the electronic wave signal is applied may be transited from the nucleus from the low energy state to the high energy state and radiate an electronic wave having a Larmor frequency. In other words, if the electronic wave signal stops being applied to the nucleus, a change in an energy level of the nucleus to which the electronic wave signal is applied occurs from high energy to low energy and thus the electronic wave having the Larmor frequency may be radiated. The fixed RF coil 2013 and the removable RF coil 2016 may receive the electronic wave signal radiated from the nuclei included in the object 10.

The fixed RF coil 2013 and the removable RF coil 2016 may be configured as one RF transceiving coil having functions of generating an electronic wave having an RF corresponding to a type of a nucleus and receiving the electronic wave radiated from the nucleus. The fixed RF coil 2013 and the removable RF coil 2016 may be respectively configured as a transmission RF coil having the function of generating the electronic wave having the RF corresponding to the type of the nucleus and a receiving RF coil having the function of receiving the electronic wave radiated from the nucleus.

The removable RF coil 2016 may include a RF coil of a part of an object including a head RF coil, a chest RF coil, a leg RF coil, a neck RF coil, a shoulder RF coil, a wrist RF coil, and an ankle RF coil.

The removable RF coil 2016 may include a display 2017 and a speaker 2018 that output notification information indicating that a RF coil connected to the MRI apparatus 2000 is for photographing the object 10 and notification information as guidance indicating that the RF coil is normally installed in the MRI apparatus 2000 or a connector is separated from the MRI apparatus 2000.

The removable RF coil 2016 may communicate with an external apparatus by wire and/or wirelessly and perform dual tune communication with the external apparatus according to a communication frequency band.

The removable RF coil 2016 may include a birdcage coil, a surface coil, and a transverse electromagnetic (TEM) coil according to a structure of a coil.

The removable RF coil 2016 may include a transmission coil, a receiving coil, and a transceiving coil according to a use of a coil.

The removable RF coil 2016 may include an RF coil of various channels such as 16 channels, 32 channels, 72 channels, and 144 channels according to a number of channels.

The gantry 2010 may further include a display 2014 and a speaker 2015 that are disposed outside the gantry 2010 or include a display (not shown) and a speaker (not shown) that are disposed inside the gantry 2010. The display 2014 and the speaker 2015 and the display and the speaker that are disposed outside and inside the gantry 2010 may be used to provide a user or the object 10 with notification information indicating that the removable RF coil 2016 installed in the MRI apparatus 2000 is for photographing the object 10 or predetermined information as guidance indicating that the removable RF coil 2016 is normally installed.

Although not shown in FIG. 20, the gantry 2010 may further include a signal transceiving unit, e.g., a signal transreceiver, that transmits a first signal to the removable RF coil 2016 and receives a second signal corresponding to the first signal from the removable RF coil 2016.

The signal control unit 2030 may control the gradient magnetic field formed inside the gantry 2010, i.e. the bore, according to a predetermined MR sequence and control transmitting and receiving of the RF signal and the MR signal.

The signal control unit 2030 may include a gradient amplifier 2032, a transceiving switch 2034, a RF transmitting unit 2036, e.g., a RF transmitter, and an MR receiving unit 2038, e.g., a MR receiver.

The gradient amplifier 2032 may drive the gradient coil 2012 included in the gantry 2010 and supply a pulse signal used to generate the gradient magnetic field under control of a gradient control unit 2074, e.g., gradient controller, to the gradient coil 2012. The pulse signal supplied from the gradient amplifier 2032 to the gradient coil 2012 is controlled and thus gradient magnetic fields in the X, Y, and Z axial directions may be combined.

The RF transmitting unit 2036 and the MR receiving unit 2038 may drive the fixed RF coil 2013 and the removable RF coil 2016. The RF transmitting unit 2036 may supply a RF pulse of the Larmor frequency to the fixed RF coil 2013 and the removable RF coil 2016. The MR receiving unit 2038 may receive the MR signal received by the fixed RF coil 2013 and the removable RF coil 2016.

The transceiving switch 2034 may adjust transmission and reception directions of the RF signal and the MR signal. For example, the RF signal may be irradiated on the object 10 by using the fixed RF coil 2013 and the removable RF coil 2016 in a transmission mode, and the MR signal may be received from the object 10 by using the fixed RF coil 2013 and the removable RF coil 2016 in a receiving mode. The transceiving switch 2034 may be controlled by using a control signal from the RF control unit 2076.

The monitoring unit 2050 may monitor or control the gantry 2010 or devices installed in the gantry 2010. The monitoring unit 2050 may include a system monitoring unit 2052, e.g., a system monitor, an object monitoring unit 2054, e.g., an object monitor, a table control unit 2056, e.g., a table controller, and a display control unit 2058, e.g., a display controller.

The system monitoring unit 2052 may monitor and control a state of the magnetostatic field, a state of the gradient magnetic field, a state of the RF signal, a state of the RF coil, a state of the table 2019, a state of a device for measuring body information of the object 10, a state of a power supply, a state of a heat exchanger, and a state of a compressor.

The object monitoring unit 2054 monitors a state of the object 10. In more detail, the object monitoring unit 2054 may include a camera for observing a movement or a location of the object 10, a respiration measurer for measuring respiration of the object 10, an electrocardiogram (ECG) measurer for measuring an EGC of the object 10, or a body temperature measurer for measuring a body temperature of the object 10. The respiration measurer, the ECG measurer, and the body temperature measurer may be removed from the gantry 2010.

The table control unit 2056 controls a movement of the table 2019 on which the object 10 is placed. The table control unit 2056 may control the movement of the table 2019 according to a sequence control of the sequence control unit 2072. For example, for moving imaging of the object 10, the table control unit 2056 may continuously or intermittently move the table 2019 according to the sequence control of the sequence control unit 2072 so that the object 10 may be photographed with a greater field of view (FOV) than that of the gantry 2010.

The display control unit 2058 controls the display 2014 and the display that are disposed outside and inside the gantry 2010. In more detail, the display control unit 2058 may control the display 2014 and the display disposed outside and inside the gantry 2010 to be turned on/off or a screen that is to be output on the display 2014 and the display. When the speaker 2015 and the speaker are disposed outside and inside the gantry 2010, the display control unit 2058 may control the speaker 2015 and the speaker to be turned on/off or sound that is to be output via the speaker 2015 and the speaker.

The system control unit 2070 may include the sequence control unit 2072 that controls a sequence of signals formed inside the gantry 2010 and a gantry control unit 2078 that controls the gantry 2010 and the devices installed in the gantry 2010.

The sequence control unit 2072 may include a gradient control unit 2074 that controls the gradient amplifier 2032 and a RF control unit 2076 that controls the RF transmitting unit 2036, the MR receiving unit 2038, and the transceiving switch 2034.

The sequence control unit 2072 may control the gradient amplifier 2032, the RF transmitting unit 2036, the MR receiving unit 2038, and the transceiving switch 2034 according to a pulse sequence received from the operating unit 2090. In this regard, the pulse sequence includes every piece of information for controlling the gradient amplifier 2032, the RF transmitting unit 2036, the MR receiving unit 2038, and the transceiving switch 2034, for example, information including the intensity of the pulse signal applied to the gradient coil 2012, a time taken to apply the pulse signal, application timing, etc.

The operating unit 2090 may instruct pulse sequence information to the system control unit 2070 and simultaneously control general operations of the MRI apparatus 2000.

The operating unit 2090 may include an input unit 2091, an output unit 2093, an image processing unit 2095, an information obtaining unit 2096, a comparison unit 2097, and a determining unit 2098.

The user may use the input unit 2091 to input object information, parameter information, a scanning condition, the pulse sequence, information regarding an image combination or a difference operation, etc. The input unit 2091 may include a keyboard, a mouse, a track ball, a sound recognition unit, a gesture recognition unit, a touch screen, etc. and may include various input devices within a scope obvious to those of ordinary skill in the art.

The output unit 2093 may output image data generated by the image processing unit 2095 or reconfiguration image data to the user. The output unit 2093 may output information for the user to operate the MRI apparatus 2000 such as user interface (UI) information, user information, or the object information. The output unit 2093 may include a speaker, a printer, a cathode-ray tube (CRT) display, a liquid crystal display (LCD) device, a plasma display panel (PDP), an organic light emitting diode (OLED) display device, a field emission display (FED) device, a light emitting display (LED) device, a vacuum fluorescent display (VFD) device, a digital light processing (DLP) display device, a primary flight display (PFD) device, a 3D display device, or a transparent display device, and include various output devices within a scope obvious to those of ordinary skill in the art.

The output unit 2093 may output the notification information indicating that the removable RF coil 2016 installed in the MRI apparatus 2000 is for photographing the object 10 or predetermined information as guidance indicating that the removable RF coil 2016 is normally installed or a connector is to be separated from the MRI apparatus 2000.

The image processing unit 2095 may process the MR signal received from the MR receiving unit 2038 and generate MR image data of the object 10.

The image processing unit 2095 may perform various signal processing such as amplification, frequency conversion, phase detection, low frequency detection, filtering, etc. on the MR signal received by the MR receiving unit 2038.

The image processing unit 2095 may allow digital data in, for example, a k space of a memory (for example, a Fourier space or a frequency space), perform 2D or 3D Fourier transformation on the digital data, and reconfigure the digital data as image data.

The image processing unit 2095 may perform synthesis processing or difference operation processing on the image data according to the need. Synthesis processing may include addition processing, maximum intensity projection (MIP) processing, etc. The image processing unit 2095 may store the reconfigured image data as well as the image data on which synthesis processing or difference operation processing is performed in a memory (not shown) or an external server.

The image processing unit 2095 may perform various signal processing applied to the MR signal in parallel. For example, the image processing unit 2095 may perform signal processing on a plurality of MR signals received by a multichannel RF coil in parallel and reconfigure the MR signals as image data.

The information obtaining unit 2096 obtains information regarding a first RF coil for photographing the object 10 and information regarding a second RF coil connected to the MRI apparatus 2000. The information obtaining unit 2096 may obtain the information regarding the first RF coil based on information including a name of a disease and a photographing part of the object 10 and receive the information regarding the first RF coil from the external server.

The information obtaining unit 2096 may obtain the information regarding the second RF coil by measuring a resistance value of a connector of the second RF coil connected to the MRI apparatus 2000 and receive the information regarding the second RF coil from the second RF coil.

The comparison unit 2097 compares the information regarding the first RF coil and the information regarding the second RF coil. The information regarding the first RF coil and the information regarding the second RF coil may include information regarding at least one of parts in which the first and second RF coils are installed, sizes, a number of channels, and use.

The determining unit 2098 may determine a state in which the removable RF coil 2016 is installed according to whether the second signal is received from the removable RF coil 2016 connected to the MRI apparatus 2000 after the signal transceiving unit included in the gantry 2010 transmits the first signal to the removable RF coil 2016.

Although the signal control unit 2030, the monitoring unit 2050, the system control unit 2070, and the operating unit 2090 are separated from each other in FIG. 20, it will be understood by those of ordinary skill in the art that functions performed by the signal control unit 2030, the monitoring unit 2050, the system control unit 2070, and the operating unit 2090 may be performed by other elements. For example, although the image processing unit 2095 converts the MR signal received by the MR receiving unit 2038 into a digital signal, the MR receiving unit 2038, the fixed RF coil 2013, or the removable RF coil 2016 may directly convert the MR signal into the digital signal.

The gantry 2010, the fixed RF coil 2013, the removable RF coil 2016, the signal control unit 2030, the monitoring unit 2050, the system control unit 2070, and the operating unit 2090 may be connected to each other by wire or wirelessly. When the gantry 2010, the fixed RF coil 2013, the removable RF coil 2016, the signal control unit 2030, the monitoring unit 2050, the system control unit 2070, and the operating unit 2090 are connected to each other wirelessly, the MRI apparatus 2000 may further include a device (not shown) for synchronizing clocks of the gantry 2010, the fixed RF coil 2013, the removable RF coil 2016, the signal control unit 2030, the monitoring unit 2050, the system control unit 2070, and the operating unit 2090. The gantry 2010, the fixed RF coil 2013, the removable RF coil 2016, the signal control unit 2030, the monitoring unit 2050, the system control unit 2070, and the operating unit 2090 may communicate with each other by using a high-speed digital interface such as low voltage differential signalling (LVDS), asynchronous serial communication such as universal asynchronous receiver transmitter (UART), a low delay type network protocol such as synchronous serial communication or a controller area network (CAN), and use various communication methods within a scope obvious to those of ordinary skill in the art.

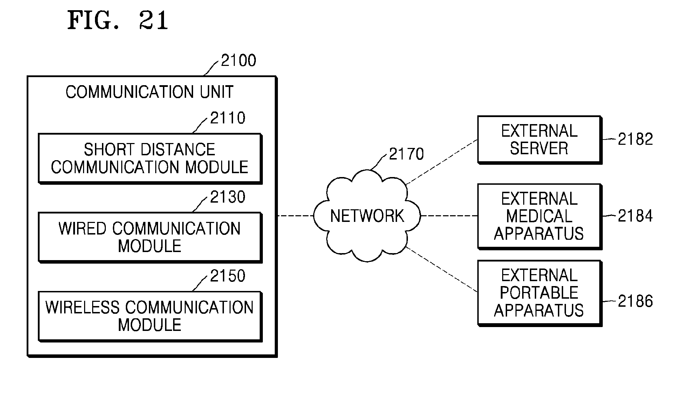

FIG. 21 is a diagram illustrating a configuration of a communication unit 2100 included in the MRI apparatus 2000 of FIG. 20.

The communication unit 2100 may be connected to at least one of the gantry 2010, the signal control unit 2030, the monitoring unit 2050, the system control unit 2070, and the operating unit 2090 of FIG. 20.

The communication unit 2100 may transmit and receive data to and from a hospital server or other medical devices of a hospital through a picture archiving and communication system (PACS) and perform data communication according to the digital imaging and communications in medicine (DICOM) standard.

As shown in FIG. 21, the communication unit 2100 may be connected to a network 2170 by wire or wirelessly, and may communicate with an external server 2182, an external medical apparatus 2184, or an external portable apparatus 2186.

The communication unit 2100 may transmit and receive data associated with a diagnosis of the object 10 over the network 2170, and may also transmit and receive a medical image photographed by the external medical apparatus 2184 such as a CT apparatus, an MRI apparatus, an X-ray apparatus, or the like. Furthermore, the communication unit 2100 may receive a diagnosis history or treatment schedule of a patient from the external server 2182, and use the received diagnosis history or treatment schedule in diagnosing the object 10. The communication unit 2100 may communicate with the external portable apparatus 2186 such as a mobile terminal, personal digital assistant (PDA), notebook computer, or the like of a doctor or a customer, in addition to the external server 2182 or the external medical apparatus 2184.

The communication unit 2100 may transmit information regarding whether the MRI apparatus 2000 is normal or not or medical image quality information to a user and receive feedback with respect to the information from the user.

The communication unit 2100 may transmit information regarding a RF coil for photographing the object 10 to at least one RF coil that is not connected to the MRI apparatus 2000.

The communication unit 2100 may include one or more elements enabling communication with an external apparatus, and for example, may include a short distance communication module 2110, e.g., a short distance communicator, a wired communication module 2130, e.g., a wired communicator, and a wireless communication module 2150, e.g., wireless communication module..

The short distance communication module 2110 denotes a module for performing short-distance communication with an apparatus within a certain distance. Short-distance communication technology according to an exemplary embodiment may include wireless LAN, Wi-Fi, Bluetooth, Zigbee, Wi-Fi direct (WFD), ultra wideband (UWB), infrared data association (IrDA), Bluetooth low energy (BLE), and near field communication (NFC), or the like, but is not limited thereto.

The wired communication module 2130 denotes a module for performing communication using an electrical signal or an optical signal. Wired communication technology may include wired communication technology using a pair cable, a coaxial cable, and an optical fiber cable, and may include other wired communication technology obvious to those of ordinary skill in the art.

The wireless communication module 2150 transmits and receives a RF signal to and from at least one of a base station, an external apparatus, and a server over a mobile network. In this regard, the RF signal may include various types of data based on transmission and reception of a voice call signal, a video call signal, or a letter/multimedia message.

The exemplary embodiments can be written as computer programs and can be implemented in general-use digital computers that execute the programs using a computer readable recording medium.

Examples of the computer readable recording medium include magnetic storage media (e.g., ROM, floppy disks, hard disks, etc.), optical recording media (e.g., CD-ROMs, or DVDs), and carrier waves (e.g., transmission through the Internet).

It should be understood that the exemplary embodiments described herein should be considered in a descriptive sense only and not for purposes of limitation. Descriptions of features or aspects within each embodiment should typically be considered as available for other similar features or aspects in other embodiments.

While one or more exemplary embodiments have been described with reference to the figures, it will be understood by those of ordinary skill in the art that various changes in form and details may be made therein without departing from the spirit and scope of the present invention as defined by the following claims.

## Claims

1. A notification method performed by using a magnetic resonance imaging (MRI) apparatus, the notification method comprising:
obtaining information regarding a first radio frequency (RF) coil for photographing an object;
obtaining information regarding a second RF coil connected to the MRI apparatus;
comparing the information regarding the first RF coil and the information regarding the second RF coil; and
outputting a notification indicating that the second RF coil corresponds to the first RF coil based on a result of the comparing.

2. The notification method of claim 1, wherein the outputting comprises: outputting the notification by using at least one of a speaker and a display that are attached to a gantry of the MRI apparatus or the second RF coil.

3. The notification method of claim 1, wherein the second RF coil comprises a wired RF coil connected to the MRI apparatus via a connector.

4. The notification method of claim 3, wherein the outputting comprises: transmitting a light signal having a color corresponding to the notification to the second RF coil via the connector and displaying the color on a display attached to the second RF coil.

5. The notification method of claim 3, wherein the obtaining of the information regarding the second RF coil comprises:
measuring a resistance value of the connector; and
obtaining the information regarding the second RF coil based on the measured resistance value.

6. The notification method of claim 3, wherein the obtaining of the information regarding the second RF coil comprises: receiving the information regarding the second RF coil from the second RF coil via the connector.

7. The notification method of claim 1, wherein the second RF coil is connected to the MRI apparatus by wire or by using a first wireless communication method,
the method further comprising: when the second RF coil does not correspond to the first RF coil, transmitting the information regarding the first RF coil to a plurality of RF coils that are not connected to the MRI apparatus by using a second wireless communication method.

8. The notification method of claim 1, wherein the information regarding the first RF coil and the information regarding the second RF coil comprise information regarding at least one of parts in which the first and second RF coils are installed, sizes, a number of channels, and use.

9. A magnetic resonance imaging (MRI) apparatus comprising:
an information obtainer configured to obtain information regarding a first radio frequency (RF) coil for photographing an object and obtain information regarding a second RF coil connected to the MRI apparatus;
a comparer configured to compare the information regarding the first RF coil and the information regarding the second RF coil, in a comparing; and
an outputter configured to output a notification indicating that the second RF coil corresponds to the first RF coil based on a result of the comparing.

10. The MRI apparatus of claim 9, wherein the outputter outputs the notification by using at least one of a speaker and a display that are attached to a gantry of the MRI apparatus or the second RF coil.

11. The MRI apparatus of claim 9, wherein the second RF coil comprises a wired RF coil connected to the MRI apparatus via a connector.

12. The MRI apparatus of claim 11, wherein the outputter transmits a light signal having a color corresponding to the notification to the second RF coil via the connector and displays the color on a display attached to the second RF coil.

13. The MRI apparatus of claim 11, wherein the information obtainer measures a resistance value of the connector and obtains the information regarding the second RF coil based on the measured resistance value.

14. The MRI apparatus of claim 11, wherein the information obtainer receives the information regarding the second RF coil from the second RF coil via the connector.

15. The MRI apparatus of claim 9, wherein the second RF coil is connected to the MRI apparatus by wire or by using a first wireless communication method,
the apparatus further comprising: when the second RF coil does not correspond to the first RF coil, a communicator configured to transmit the information regarding the first RF coil to a plurality of RF coils that are not connected to the MRI apparatus by using a second wireless communication method.
